# EUROPEAN PATENT SPECIFICATION

(11) **EP 2 252 154 B1**
(45) Date of publication and mention of the grant of the patent: **27.01.2016**
(21) Application number: 09710022.6
(22) Date of filing: 13.02.2009
(51) Int. Cl.: A01N 65/00, A61K 8/49, A61K 8/97, A61Q 17/04

(54) **METHOD FOR MINIMIZING THE CYTOTOXICITY OF THE EFFECTIVE AGENT IN A COMPOSITION THAT IS ADMINISTERED TO A MAMMAL OR GETS INTO CONTACT WITH A MAMMAL**
VERFAHREN ZUR MINIMIERUNG DER CYTOTOXIZITÄT DES WIRKSTOFFS IN EINER ZUSAMMENSETZUNG, DIE AN EIN SÄUGETIER VERABREICHT WIRD ODER MIT EINEM SÄUGETIER IN KONTAKT KOMMT
PROCÉDÉ POUR RÉDUIRE LA CYTOTOXICITÉ DE L'AGENT ACTIF DANS UNE COMPOSITION ADMINISTRÉE À UN MAMMIFÈRE OU VENANT EN CONTACT AVEC UN MAMMIFÈRE

(30) Priority: 14.02.2008 FI 20080112
(43) Date of publication of application: 24.11.2010
(73) Proprietor: Oy Granula AB LTD, 48101 Kotka (FI)
(72) Inventor: AHLNÄS, Thomas, FI-48130 Kotka (FI)
(74) Representative: Berggren Oy Ab
(86) International application number: PCT/FI2009/050115
(87) International publication number: WO 2009/101261

(56) References cited:
- WO-A-2004/000304
- WO-A-2005/047423
- WO-A-2007/096088
- WO-A-2007/096089
- WO-A-2007/096090
- WO-A-2008/020112
- VALIMAA ET AL: "Antimicrobial and cytotoxic knotwood extracts and related pure compounds and their effects on food-associated microorganisms" INTERNATIONAL JOURNAL OF FOOD MICROBIOLOGY, ELSEVIER SCIENCE PUBLISHERS, AMSTERDAM, NL, vol. 115, no. 2, 12 March 2007 (2007-03-12), pages 235-243, XP005921921 ISSN: 0168-1605 cited in the application
- BJARNE HOLMBOM ET AL: "Knots in trees A new rich source of lignans" PHYTOCHEMISTRY REVIEWS, KLUWER, NL, vol. 2, no. 3, 1 October 2003 (2003-10-01), pages 331-340, XP008104241 ISSN: 1568-7767 [retrieved on 2004-11-08]

## Description

The invention relates to the production of a composition according to claim 1.

Sunlight contains both UVA radiation, with a wavelength of 320-400 nm, and UVB radiation, with a wavelength of 280-320 nm. Both UVA and UVB radiation have detrimental cytotoxic effects on skin cells, but with different effect mechanisms. UVB radiation directly induces mutations and damages in the DNA of skin cells. As for UVA radiation, it induces the creation of reactive free radicals on the skin surface from coumarin-type compounds. Thereafter said free radicals can damage skin cells, thus predisposing the skin to various cancer diseases. The effect of UVB is inhibited by protecting the skin with sun protective compositions containing UV filtering agent that transfers UVB radiation into a form that is harmless to skin cells, such as heat or long-wave radiation. The effect of UVA radiation is inhibited by UV filters (UV protection agents) that are capable of trapping free radicals induced by sunlight.

Both inorganic and organic compounds are used in sun protective compositions as UV protection agents, to prevent the detrimental effect on skin cells of UV radiation contained in sunlight. They either absorb and reflect UV radiation within a relatively narrow wavelength range, or alternatively capture free radicals induced by UV radiation, such as oxygen, hydroxyl or peroxide radicals.

Inorganic UV filters (below also inorganic UV protection agents) are finely divided (10-100 nm) inorganic mineral pigments, such as TiO₂, ZnO and FeₓO_{y}, which reflect or absorb UV radiation.

Among the compounds used in organic sun protective compositions and serving mainly as UVB protection agents, absorbing UVB radiation, are for example octyl triazine, urocanic acid, octyl methoxy cinnamate, methylbenzylidene camphor, 3-benzylidene sulphonic acid and PABA i.e. octyl dimethyl, 4-aminobenzoic acid (para-aminobenzoic acid).

Typical UVA radiation inhibitors used in sun protective compositions, functioning by the free radical trapping mechanism, are organic aromatic compounds containing conjugated carbonyl groups. Among these are for example benzophenon-3, benzophenon-4 butyl methoxydibenzoylmethane and terephtalyliden sulphonic acid. Said aromatic compounds protect the DNA of skin cells by preventing conjugated DNA double bonds from absorbing UV radiation both within the UVA range (320-360 nm) and also within the UVB range (280-320 nm).

At present, there are also available organic UV protection agents that are capable of absorbing UV radiation both within the UVA range and the UVB range. This kind of agent is for example MBBT.

Antioxidants are often added in sun protective compositions in order to prevent the oxidation of the compounds contained in the composition and to capture the possibly created oxygenous reactive compounds and free radicals. Also in foodstuffs there are used antioxidants that are capable of neutralizing, i.e. trapping oxygenous free radicals created through the auto-oxidation of foodstuffs, such as peroxide radicals. Thus they slow down the auto-oxidation process of food, preventing changes in the color, smell and taste of food. Among these antioxidants, there are natural vitamins such as A, B, C, D and E vitamins, and synthetic vitamins such as the above mentioned analog of vitamin B, i.e. para-aminobenzoic acid, the synthetic phenolic analog of vitamin E, BHT, i.e. 2,6-(bis)-(1.1-dimethylethyl)-4-methylphenyl (butylated hydroxy toluene) and BHA (butylated hydroxy anisole, which is a mixture of two isomeric organic compounds, containing 2-tert-butyl-4-hydroxy anisole and 3-tert-butyl-4-hydroxy anisole. The antioxidants used in foodstuffs and sun protective compositions may in certain conditions be cytotoxic to the human organism. Among these, there are also BHT (foodstuff additive E321) and BHA, which means that their use is restricted, and they cannot be sufficiently added to the target of usage in order to ensure their antioxidative effect.

Certain organic UV protection agents or inorganic mineral compounds used for absorbing UV radiation may, however, spontaneously release free radicals owing to the effect of UV radiation, or they may induce the creation of free radicals in the target of usage. Among free radicals, let us mention for example hydroxyl radicals, peroxide radicals, unimolecular oxygen, and temporary lipid radicals. When penetrating to the human system, free radicals may cause cytotoxic exposure.

Consequently, compounds used in sun protection agents may, owing to the effect of UV radiation, spontaneously release free radicals. As was pointed out above, in sun protective compositions there are generally used inorganic UV protection agents TiO₂ and ZnO, CeO₂, which absorb UV radiation within a wide wavelength range. However, inorganic UV filters have a strong tendency to be agglomerated owing to the effect of electrostatic forces, which means that their efficiency is drastically reduced. Therefore said finely divided inorganic pigments must be coated with silica or aluminum compounds and dispersed in a solvent, such as dispersion oil, for example in isononyl-isononanoate or dimethicone, in the presence of suitable surface active agents. Coated inorganic UV-filter mediums may themselves turn cytotoxic; in aqueous conditions, they may induce the creation of hydroxyl radicals on the skin, in case their protective coating layer is worn off. They may also themselves turn into reactive radicals, in case there are holes in their coating, or if the coating is worn off.

In the course of time, several organic UV protection agents, such as PABA, OMC and Octocrylene, gradually penetrate into the skin. In case the sun protective composition is not reapplied on the skin, or if the layer applied on the skin is otherwise worn off, there are created monoatomic reactive oxygen radicals owing to the effect of UV light. It has also been found that benzophenon-3, a UV filter generally used in sun protection agents, also penetrates into the system through the skin, which means that it may itself induce the creation of free radicals deeper in the system.

It is often necessary to add microbicidic agents into cosmetic compositions and technical solvents and cleaning agents as well as industrially used solvents. Microbicidic agents may be cytotoxic or otherwise detrimental to the system when getting into contact with the skin.

The object of the invention is to eliminate the above described drawbacks of the prior art.

Thus, the principal object of the invention is to realize a composition and a method for producing said composition, wherein the unwanted properties of the effective agent contained in the composition, such as UV protection agent, are reduced, or the action of the composition has changed. Unwanted properties are for example cytotoxicity of the agent, as well as its tendency to form or induce free radicals on the skin, skin irritation. One example of the changing of the action of the composition is that the in vitro protective factor of the composition containing UV protection agent has remained the same, while its protective factor in vivo has raised. Further, the principal object of the invention is to realize compositions or semifinished compositions used in the production of said compositions, where the effective agent or part of it is obtained from nature.

A further object of the invention is to reduce the detrimental properties of the effective agent contained in the composition by using a mixture that has good availability and low manufacturing expenses.

Free radical here refers to a molecule or an atom that has unpaired electrons in its electron shell. Typical free radicals are oxygen radical, hydroxyl radical and peroxyl radicals as well as superoxide radicals, but also for example lipid radicals. Further, for instance titanium oxide can be formed into a reactive free radical, in case it is tuned to a higher energy state owing to the effect of UV radiation. A compound is called free radical capturer, in case it is capable of inhibiting the creation of free radicals, or their function. In case the compound is capable of inhibiting the creation of oxygenous free radicals and their effects, the compound is called antioxidant.

### General description of invention

More precisely, the invention relates to a method for producing a cosmetic composition, food industry composition, animal feed composition, technical composition or packing material composition that is administered to a mammal or gets into contact with a mammal. The composition to be prepared contains a carrier agent such as a solvent, with either hydrophobic or hydrophilic nature, selected according to the target of usage, as well as possible additives, such as a surface active agent, as well as a first effective agent, which is selected among the following group: micro-organisms growth inhibiting agent, UV protection agent, antioxidant, or capturer of free radicals.

Now in the composition, there is included a carrier agent conventionally used in a cosmetic composition, food industry composition, animal feed composition, technical composition or packing material composition, possible auxiliary agents and possible surface active agent, as well as a first effective agent, which is selected among the following group: anti-micro-organistic agent, UV protection agent, antioxidant, or capturer of free radicals. Further, in the composition, there is included a compound mixture for modifying the properties of the first effective agent, which compound mixture is obtained by pulverizing wood material from two different wood species and/or by extracting the possibly pulverized wood material from two different wood species, so that said mixture contains at least two different compounds selected from among the following group: lignans according to general formulas IA and IB, stilbenes according to general formula II, juvabiones according to general formula III, flavonoids according to general formula IV and betulin and its derivatives (betulinic acid, betuloinic acid or betulonic acid), said mixture also containing oligomers of said polyphenolic compounds, providing, however, that the compound mixture contains lignans according to formulas IA or IB, or their ether or ester derivatives or stereoisomers, particularly 7-hydroxymatairesinol or secoisolariciresinol, cycloisolariciresinol, anhydrosecoisolariciresinol, α-conidendrin, α-conidendric acid, isohydroxymatairesinol, or their ether and ester derivatives and stereoisomers for roughly 50-99.9 wt%; stilbenes according to formula II, particularly pinosylvin or its ester or ether derivatives for roughly 0.1 - 70 wt%; oligomers of lignans according to formula IA or IB, of stilbenes according to formula II, of juvabiones according to formula III or of flavonoids according to formula IV for roughly 1 - 31 wt%; providing, however, that the mixture contains at least one compound, selected from among the following group: 7-hydroxymatairesinol, secoisolariciresinol, cycloisolariciresinol, anhydrosecoisolariciresinol, α-conidendrin, α-conidendric acid, isohydroxymatairesinol and stilbenes according to formula II, as well as their ether and ester derivatives and stereoisomers.

In the composition, there is included said compound mixture for 0.1 - 5 wt% of the total weight of said composition, providing, however, that with said content, the compounds added in the composition do not irritate the skin in a so-called single patch test, and that the cytotoxicity of said compounds, measured as the cytotoxicity of compounds dissolved in ethanol with a HaCat cell culture after 24 incubations, is lower than the cytotoxicity of 0.02 - 0.1 wt% BHT dissolved in ethanol in the same incubation conditions, preferably lower than the cytotoxicity of 0.01 - 0.05 wt% BHT dissolved in ethanol in the same incubation conditions, in addition to which the mixture within said content range has properties for modifying the effect of the first effective agent in the target of usage.

Here the term stereoisomers refers to compound diastereomers and to mixtures of different diastereomers, to pure enantiomers and rasemic mixtures of enantiomers.

The invention is based on the surprising observation that sufficient attention has previously not been paid to the level of overall cytotoxicity of the effective agents in cosmetic materials, foodstuffs, animal feed and packing materials, but each effective agent to be included in the composition, such as antioxidant, has been added to an extent that its maximum cytotoxicity by any means has allowed. With this observation in mind, the applicant has attempted to achieve compositions where the overall cytotoxicity of the different components of the effective agent is brought as low as possible, while the effect of the effective agent remains the same.

In the present invention, the optimizing of the overall cytotoxicity is realized by using, in addition to the first effective agent, also a second effective agent, which is a compound mixture that is obtained from wood material by extracting and/or pulverizing and that is not essentially refined. The mutual ratio between the phenolic compounds contained in said compound mixture, as well as the compounds contained in the mixture, can change depending on the effects required in the target of usage. The overall cytotoxicity of the compound mixture must, however, be as low as possible and remain below certain limits in comparison with the cytotoxicity of BHT, irrespective of the phenolic compounds contained in the mixture. The effective power and effective profile of the compound mixture are adjusted according to the target of usage of the composition, so that by using the compound mixture, the harmful properties of the effective agent, such as its own cytotoxicity, can be remarkably reduced in the target of usage, such as on the skin or hairs of a mammal, for instance by reducing the quantity of the first effective agent from the conventionally applied level. At the same time, the effective power and effective profile of the first effective agent are maintained the same or even expanded and modified by using a compound mixture that has, in addition to a low overall cytotoxicity, also a similar type of effective profile and effective power in the target of usage as the first effective agent. Apart from an effective profile similar to the first effective agent, the compound mixture often has other advantageous properties in the target of usage, so that by using this type of effective agent composed of a compound mixture and a first effective agent, the effective profile of the first effective agent contained in the composition can be modified.

The production method of the compound mixture is simple, and it is amply available from the pulping processes of wood processing industry, so that the replacing of the first effective agent by the compound mixture is economically feasible. Moreover, the compound mixture according to the invention has several other advantageous properties that its separate individual components do not necessarily have as such. The mixture according to the invention does not irritate the skin even with high contents, is physiologically well tolerated and has a low cytotoxicity. The lignans contained by the compound mixture are semipolar by nature, wherefore they can be included both in the aqueous and oil phases of the compositions.

In this connection, the modification of the effective profile of the first effective agent means that by means of the compound mixture, there are obtained additional properties in the composition, which properties are similar to the basic properties of the first effective agent. Among such additional properties are improvements in the UVA or UVB radiation absorbing efficiency of the UV protection agent, an increase in the in vivo sun protective factor (SPF) of the UV protection agent, improvements in the efficiency of antioxidants in trapping free radicals, etc.

As for reducing the harmful properties of the first effective agent, we mean that the unwanted properties of the first effective agent, such as cytotoxicity or skin irritation, tendency to form or induce free radicals in the environment of usage, etc., can be reduced.

The composition can be a cosmetic composition, a food industry composition, an animal feed composition, a technical composition, or a packing material.

The term 'cosmetic composition' here refers to compositions meant for the treatment of the skin, teeth, hair and body hair of mammals. In these, the carrier agent is a semisolid material such as cream, gel or paste; solid material such as solid foam; heterogeneous solid material or powder; liquid material such as homogeneous solution; colloidal as a dispersion or suspension; a microemulsion, a nanoemulsion, or a gaseous material such as aerosol or mist.

Food industry compositions here refer to both individual foodstuffs and foodstuff products. In this sense, food industry compositions are for example fruits, vegetables etc. individual foodstuffs, but also food industry products and semifinished products, in the preservation of which there are needed anti-micro-organistic agents.

The packing material composition is a composition where the carrier agent is a packing material that is impregnated by a compound mixture according to the invention, in which there is spread the compound mixture according to the invention.

The technical composition is an agent where the carrier is a solvent used in cleaning, a cleaning device such as a cleaning cloth, solid material such as a powder used as a surface treatment agent; or the carrier agent is a solution meant for cleaning the body of a mammal. In the carrier, there is impregnated or otherwise included in the compound mixture according to the invention.

The manufacturing of the compound mixture is simple, and the mixture is amply available from wood industry pulping processes, so that the replacing of the effective agent by said mixture also is economically beneficial. The compound mixture according to the invention also has several other advantageous properties that its individual components do not necessarily have, so that by using the mixture, new properties can be obtained in the manufactured compositions. The compound mixture is advantageously an unpurified powder ground of wood material, or a compound mixture extracted from wood material in an extraction solution, said compound mixture forming a homogeneous solution, suspension or dispersion with the extraction solution. Even more preferably, the compound mixture is an unpurified lignan mixture and/or stilbene mixture placed in a liquid, said mixture containing at least two different lignan and/or stilbene compounds. The mixture is advantageously obtained by extracting wood material from two different wood species in an alcohol-based solution. Preferably the wood material is obtained from wood knot material or stemwood material from adjacent to knots. Here the term unpurified compound mixture refers to a mixture that is obtained by pulverizing and/or extracting wood or plant material, which mixture has thereafter not been subjected to any such chemical or physical cleaning operations by which one of the mixture compounds would be completely removed from the mixture. Typical cleaning operations in this sense are chromatography or crystallization of certain compounds from the solution. Instead, the mixture may be subjected to such chemical treatments by which the content of one of the mixture compounds is adjusted in relation to other compounds contained in the mixture. This kind of operation is for example extraction by which the mutual contents of the compounds contained in the solution are adjusted with respect to each other, but by which the compounds are not completely removed. The polyphenolic compounds contained in the mixture may also be transformed into simple derivatives such as their esters, or into completely other stilbene/lignan/flavonoid compounds, when the pH of the extract solution is changed. The mixture to be included in the composition according to the invention, which mixture has free radical capturing properties, contains preferably at least two different phenolic compounds selected from the following groups:
Lignans:
   - matairesinol, hydroxymatairesinol, oxomatairesinol, didemethyl matairesinol, didemethyl matairesinol, isohydroxymatairesinol, epi-isohydroxymatairesinol and their stereoisomers, among which particularly let us point out hydroxymatairesinol stereoisomers 7S, 8R, 8'R-hydroxymatairesinol and 7R, 8R, 8'R-allohydroxymatairesinol, and their stereoisomers and ester or ether derivatives,
   - secoisolariciresinol, isolariciresinol, lariciresinol, pinoresinol, dimethyl secoisolariciresinol, 7-hydroxysecoisolariciresinol, cyclolariciresinol, cycloisolariciresinol, anhydrosecoisolariciresinol, alpha-conidendrin, alpha-conindendric acid, isohydroxymatairesinol and their stereoisomers as well as their ester or ether derivatives,
   - nortrachelogenin and its stereoisomers and ester or ether derivatives,
   - enterolactone and its stereoisomers and ester or ether derivatives,
   - conidendrin, alpha-conindenrin and their stereoisomers as well as ester or ether derivatives,
   - lignan A and its stereoisomers and ester or ether derivatives,
   - liovile and its stereoisomers and ester or ether derivatives;
Juvabiones:
   - juvabiones and their stereoisomers and ester or ether derivatives;
Stilbenes:
   - pinosylvin, dihydropinosylvin, pinosylvin monomethyl ether, dihydropinosylvin monomethyl ether, resveratrol, astringin, isorhapontine, and their stereoisomers and ester or ether derivatives;
Flavonoids:
   - pinosembrin, catechin, pinobanxin, kaempferol, dihydrokaempferol, taxifolin, naringenin, teracasidine, ketoteracasidine, isoteracasidine, melacasidine, isomelacasidine and their stereoisomers and ester or ether derivatives,
   - betulin, betulinic acid, betuloinic acid or betulonic acid and their stereoisomers and esterized forms,
   - as well as the glycosidized forms of these phenolic compounds, and their oligomers such as trimers and tetramers. These oligomers are here called oligolignans in case they are oligomers of lignans, stilbenes or juvabiones; free lignans and stilbenes are dimers, having 2 phenylpropane units coupled together by beta-beta bonds, and their oligolignans have 3 - 6 phenyl propane units (C₆C₃) coupled together by beta-beta bonds.

In this connection let us point out that compounds called lignans are generally (poly)phenolic compounds obtained from wood and plants, having 2 phenyl propane units coupled together by beta-beta bonds (IUPAC, 2000), but in this application it has been considered necessary to distinguish lignans, stilbenes and juvabiones from each other owing to their different microbiological effects.

Lignans proper here refers to compounds according to the general formula IA and IB (Appendix).

Now, in formula IA:
R1 or R2 denote, irrespective of each other, residue selected from the groups hydrogen, OH or =O,
or either one of the residues R1 or R2 denotes the oxygen atom -O- bound to carbons 9 and 9', and now forms with carbons 8, 8', 9, 9' a 5-membered oxygenous heterocyclic ring C,
R3 denotes hydrogen or residue selected from the group OH, =O, or it forms a bond to the carbon 6, so that the carbons 6, 1, 7, 8, 8', 7' form a cyclohexane ring that is condensed with the phenyl ring A, and possibly also a ring with C,
R4 denotes hydrogen or methyl,
R5 denotes hydrogen or residue selected from the groups OH and OCH₃,
R6 denotes hydrogen or hydroxy,
R7 and R8 denote, irrespective of each other, hydrogen or residue selected from the groups OH and OCH₃.

Advantageous lignans according to formula 1A are:
7-hydroxymatairesinol (R1 denotes group =O, R2 denotes the oxygen atom pertaining to the hetero ring C, R4= CH₃, R7= OCH₃, R3=R5=R8=OH, R6=H, R9=H),
matairesinol (R1 denotes group =O, R2 denotes the oxygen atom pertaining to the hetero ring C, R3=R6=R9=H; R4= CH₃, R7= OCH₃, R5=R8=OH),
oxomatairesinol, which differs from hydroxymatairesinol in that R3 denotes group =O,
didemethyl matairesinol, which differs from hydroxymatairesinol in that R4 and R3 denote hydrogen,
isohydroxymatairesinol,
alpha-conidendrin (R1 denotes group =O, R2 denotes the oxygen atom pertaining to the hetero ring C, R4= CH₃, R7= OCH₃, R8=OH, R6=H, R3 is a bond to the carbon 6, R9=H),
alpha-conidendric acid (R4= CH₃, R7= OCH₃, R5=R8=OH, R6 denotes group =O, R1=R2=OH, R3 is a bond to the carbon 6, so that the carbons 6, 1, 7, 8, 8', 7' form a cyclohexane ring D that is condensed with the phenyl ring A, R9=H),
liovile (R4= CH₃, R7= OCH₃, R3=R5=R6=R8=OH, R1=H, R2 denotes the oxygen atom pertaining to the hetero ring C, R3 denotes a bond to the carbon 6, R9=H),
secoisolariciresinol (R1=R2= OH, R3=H, R4= CH₃, R7= OCH₃, R5=R8=OH, R6=R9=H),
dimethyl secoisolariciresinol, which differs from secoisolariciresinol in that R5 and R8 are methoxies,
isolariciresinol (R1=R2=OH, R4= CH₃, R7= OCH₃, R5=R8=OH, R6=H, R3 is a bond to the carbon 6, so that the carbons 6, 1, 7, 8, 8', 7' form a cyclohexane ring D, which is condensed with the phenyl ring A, R9=H),
cyclolariciresinol (R1=R2= OH, R3= a bond to the carbon 6, so that the carbons 6, 1, 7, 8, 8', 7' form a cyclohexane ring D, which is condensed with the phenyl ring A, H, R4= CH₃, R7= OCH₃, R5=R8=OH, R6=R9=H),
nortrachelogenin (R1 denotes group =O, R2 denotes the oxygen atom pertaining to the hetero ring C, R3=R6=H, R4=CH₃, R7=OCH₃, R5=R8=OH, R9=OH),

In Formula IB:
R10 denotes hydrogen or hydroxy
R11 denotes hydroxy or oxygen, which is bound by a bond to the carbons 7 and 9', forming now an oxygenous non-aromatic 5-membered heterocyclic ring (tetrahydrofuran) F with the carbons 7, 8, 8', 9', which ring is condensed in the hetero ring E (tetrahydrofuran) at the carbons 8, 8',
R12 denotes hydrogen or methyl,
R13 denotes hydrogen or methoxy,
R14 denotes hydrogen or methoxy,
R30 denotes hydrogen or group =O.

Advantageous compounds according to formula IB are: pinoresinol (R13=R14= R12=R10=R30=H, and R11 is oxygen in the hetero ring F); isohydroxymatairesinol (R12=R13=R14=R10=H, R11=OH, R30 denotes group =O); lariciresinol (R12=R13=R14=R10=R30=H, R11=OH); and lignan A (R10= R11=OH, R12=R13=R14=R30=H).

Stilbenes in turn refer to compounds according to the general formula II (Appendix),
where R15 denotes hydrogen or hydroxy
R16 denotes residue selected from the groups H, OH, OCH₃,
R17 denotes residue selected from the groups OH or OCH₃,
R18 and R19 denote, irrespective of each other, hydrogen or hydroxy,
R20 denotes residue selected from the groups hydrogen, OGlu
A few advantageous stilbenes according to formula II are pinosylvin (R18=R19=R20=R15=H, R16=R17=OH), monomethyl ether of pinosylvin (R18=R19=R20=R15=H, R16=OCH₃, R17=OH), dihydropinosylvin (R18=R19=R20=R15=H, R16=R17=OH, the phenyl elements bonding ethenyl residue is hydrated to ethyl), resveratrol (R16=R17=R19=OH, R18=R20=H), astringin (R15=R17=OH, R16=R19=H, R18=OH, R20=OGlu and isorhapontine (R16=R19=H, R17=OCH₃,R15, R18=OH, R20=OGlu).
Juvabiones refer to compounds according to formula III (Appendix).

Flavonoids refer to compounds according to general formula IV (Appendix), where
R21 denotes residue selected from the groups H, OH,
R22 denotes residue selected from the groups H, OH, =O,
R23 denotes residue selected from the groups H, OH
R24, R25, R26 denote, irrespective of each other, hydrogen or hydroxy,
R26 and R27 denote, irrespective of each other, hydrogen or hydroxy.

Among advantageous compounds according to formula IV are dihydromyricetin (R27=H, R21=R23=R24=R25=R26=R28=OH, R22 is oxo group), taxifolin (R24=R27=H, R21=R23=R25=R26=R28=OH, R22 is oxo group), dihydrokaempferol (R24=R26=R27=H, R21=R23=R25=R28=OH, R22 is oxo group), catechin (R24=R26=R27=H, R21=R23=R25=R26=R28=OH, R22 is hydrogen), naringenin (R23=R24=R26=R27=H, R21=R25=R28=OH, R22 is oxo group), kaempferol (R24=R26=R27=H, R21=R23=R25=R28=OH, R22 is oxo group), teracasidine (R21=R24=H, R22=R23=R25=R26=R27=R28=OH, ketoteracasidine (R21=R24=R26=H, R23=R25=R27=R28=OH, R22 is oxo group), isoteracasidine (R21=R24=R26=H, R22=R23=R25=R27=R28=OH), melacasidine (R21=R24= H, R22=R23=R25=R26=R27=R28=OH), isomelacasidine (R21=R24=H, R22=R23=R25=R26=R27=R28=OH), pinobanxin (R24=R25=R26=R27=H, R21=R23=R28=OH, R22 is oxo group) and pinosembrin (R23=R24=R25=R26=R27=H, R21=R28=OH, R22 is oxo group).

Betulin, by systematic name (IUPAC) lup-20(29)-ene-3β,28-diol, and its derivatives refer to compounds according to formula 1E. In the formula 1E, betulonic acid is compound 2, and betulinic acid is compound 3. Betulin is compound 1. Compounds 2 and 3 are obtained by oxidizing betulin 1 into compound 2, and by reducing compound 2 into compound 3 (US 6.280.778). Betuloinic acid is a derivative of betulonic acid.

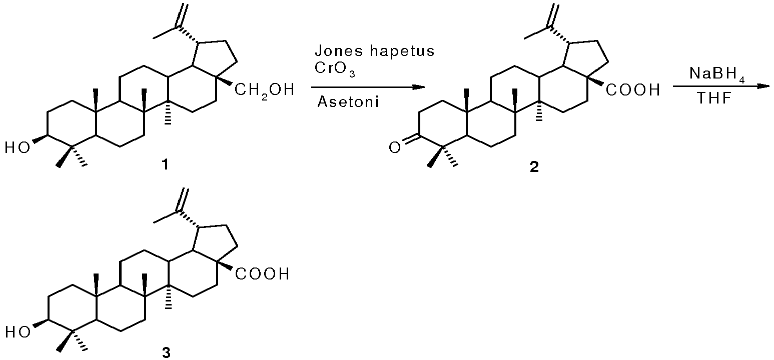

Exemplary oligolignans are beta bound guaiacyl ethers of lignans and stilbenes trimeric so-called sesquilignans) and coumarates such as secoisolariciresinol guaiacyl glycerol ether, nortrachelogenin guaiacyl glycerol ether, hydroxymatairesinol guaiacyl glycerol ether, lariciresinol guaiacyl glycerol ether, liovile guaiacyl glycerol ethers, conidendrin guaiacyl glycerol ether, pinoresinol guaiacyl glycerol ether, lariciresinol coumarate and secoisolariciresinol coumarate (Willför et al, Holzforchnung, Vol 58, 3435 -354, 2004) and dilignans such as 5-5-bis-secoisolariciresinol, 5-5-bis-isolariciresinol, 5-5-bis-lariciresinol.

Depending on the pH, phenolic compounds occur in the compositions either as free, esterized or etherized forms, wherefore also said ester and ether derivatives belong within the scope of the invention.

The phenolic compounds contained by the mixture according to the invention are well tolerated by mammals, they have a low cytotoxicity and they neither spontaneously form nor induce free radicals. Below, in Table 1, we have compared the cytotoxicity of the polyphenolic mixture according to the invention with the cytotoxicity of an antioxidant (BHT) that is widely used in the food and cosmetic industry.

Thus, most of the compound mixtures according to the invention are relatively mild anti-micro-organistic agents, but this is compensated in that owing to their non-toxic nature, they can be used in remarkably higher quantities.

One of the most important properties of the compound mixtures according to the invention is their minimal penetration to skin, wherefore skin irritation does not occur when using them, as opposite to the conventionally used anti-micro-organistic compounds. The applicant has studied the skin irritation of the compound mixtures according to the invention in a so-called single patch test and found out that they do not irritate the skin with 0.1 wt% contents, and not even with 1 wt% contents; often even 5 wt% contents can be used without excessive skin irritation.

The low cytotoxicity of the compound mixtures according to the invention, as combined to a minimal skin irritation, ensures that the compound mixture according to the invention can be used in sufficiently large quantities, particularly in cosmetic compositions. Thus, by using the compound mixtures, the anti-micro-organistic effect of the agent used as the first anti-micro-organistic agent can be expanded or increased, and at the same time the cytotoxicity of the effective agents as well as their skin irritation can be maintained sufficiently low.

When the first effective agent to be included in the composition is an anti-micro-organistic agent, in the composition is preferably included a compound mixture that has anti-micro-organistic properties against at least the same micro-organisms as the first effective agent.

One of the most important properties in compositions provided with a compound mixture according to the invention is that the mixture of phenolic compounds present in the compositions inhibits on a wide scale the growth of anti-microbial agents, when it is added in the composition for 0.1-5 wt%. Generally the amount of synthetic widely anti-microbial agents that can be added to compositions is only roughly 0.01-0.03 wt%, owing to their high cytotoxicity, in case the compositions get into contact with mammal skin at some stage of their usage life. As for Gram-negative bacteria, a mixture of phenolic compounds has a growth inhibiting effect at least against E. coli, Ps. aeruginosa, Ps. putida, and Kl. pneimoniae; as for Gram-positive bacteria, it has a growth-inhibiting effect at least against S. aureus; as for yeasts, it has a growth-inhibiting effect at least against M. furfur and C. albicans, and as for fungi, a growth-inhibiting effect at least against A. niger.

A particularly surprising feature in the invention is that although it has been found out that several pure lignan, stilbene or flavonoid compounds, or knot extracts obtained from trees containing abundantly such lignans, stilbenes or flavonoids, have a limited effect against the growth of micro-organisms, yeasts or fungi, it has not been verified that they should have a wide-scale anti-micro-organistic effect, and they have not been found effective for example against certain important Gram-positive bacteria such as S. aureus (cf. e.g. Välimaa et al., International J. of Microbiology, 115 (2007) 235-243). Moreover, it has earlier been shown that stilbene-bearing raw extracts and solutions containing refined stilbenes are relatively cytotoxic (e.g. International J. Food Microbiology, 115 (2007) 235-243), which does not encourage a man skilled in the art to use these extracts in cosmetic products.

The wide-scale anti-micro-organistic effect of the mixtures containing phenolic compounds used in the invention, combined with their low cytotoxicity, is a surprising feature, because in the raw extracts obtained from knotty knotwood described in the prior art, their anti-micro-organistic effect has not been verified as particularly wide-scale, not even for raw extracts containing stilbenes.By using the microbial growth inhibiting compound mixture according to the invention, it is often possible to modify the harmful properties of other anti-micro-organistic agents, such as microbicidic and bacteriostatic agents contained in the composition, by trapping the free radicals created during their decomposition, which reduces the cytotoxicity and skin irritation of these agents.

A new feature with compositions realized by means of compound mixtures is their improved in vivo sun protection factor SFP, in comparison with measurements in vitro.

By adding the compound mixture according to the invention to cosmetic and food industry compositions, with an antioxidant such as a vitamin present as the first effective agent, the effective profile of antioxidants, as well as their effective time, can be adjusted, and their cytotoxicity reduced.

Yet another way to use the compound mixtures according to the invention is to add them to the coating of finely divided inorganic UV protection agents. In that case the compound mixture has free radical neutralizing effects, which is an effective way to prevent the unwanted effects of free radicals created of the inorganic UV protection agent, such as the skin irritation caused by them.

Among others, the anti-micro-organistic compound mixture according to the invention can be used in cosmetic compositions and their semifinished compositions, such as sun protection compositions and in semifinished products used in the manufacturing of sun protection compositions. The mixture can also be used in so-called commercial solvents, such as surface treatment agents, solvents used in the cleaning branch solvents etc.

### Description of preferred embodiments of invention

The compound mixture according to the invention can advantageously be used in sun protection compositions and in semifinished products used in the manufacturing of sun protection compositions.

As was already pointed out, the organic UV protection agents used in sun protection compositions may themselves induce the creation of free radicals, in the topmost layer of the sun protection composition applied on the skin is worn off, or in case the UV protection agents have access to be absorbed in the skin, and a new layer of sun protection composition is not applied on the skin. Organic UV protection agents function either by absorbing UV radiation and by transforming it to a longer-wave radiation that is less harmful to skin cells (for example into heat), or then they capture the free radicals created on the skin owing to the effect of the UV radiation. The mixtures according to the invention, containing phenolic compounds, have free radical capturing properties, wherefore they can be used for inhibiting the creation of free radicals from organic UV protection agents, when they are exposed to UV radiation. By adding a mixture according to the invention, containing (poly)phenolic compounds, in sun protection compositions containing organic UV protection agents, or in semifinished products used in the manufacturing thereof, containing organic UV protection agents, it is possible to prevent the organic UV protection agents from themselves turning cytotoxic to the system. A mixture according to the invention, containing phenolic compounds, also has UV radiation absorbing properties itself, the mixture does not spontaneously create reactive free radicals, and the manufacturing expenses of the mixture are low and availability good, wherefore it can advantageously be used for replacing part of the expensive organic UV protection agents. The effective mechanism of the polyphenols contained in the mixture according to the invention would appear to be mainly based on that the phenolic compounds contained in the mixture go through an automatic oxidation-reduction reaction after neutralizing the free radicals. This automatic oxidation-reduction reaction ends in the formation of stabile dimers. A secondary effect of the mixture according to the invention is in that they are capable of absorbing UV radiation and of preventing the action of free radical creating enzymes, and/or of preventing the action of metal ions catalyzing the creation of free radicals or of decomposing hyperoxides. Mixtures according to the invention can be used, among others, with the following protection agents in sun protection compositions or in semifinished products used in the manufacturing of sun protection compositions:
- Avobenzon (BMDN i.e. 1-(4-methoxyphenyl)-3-(4-tert.- butylphenyl)propane-1,3-dione, octyldimethyl-4-aminobenzoic acid. Avobenzon absorbs UVA radiation in a wide spectrum. Avobenzon is often used in sun protection compositions together with titanium oxide in order to expand the effective spectrum thereof. Because Avobenzon is gradually decomposed owing to the effect of sunlight, various photo stabilizers are often used for stabilizing it, among them Octocrylene, as well as triazine derivatives (bis-ethylhexyl oxiphenyl triazine or methylene-bis-benzotriazolyl) known by the trade names Tinosorb® S and Tinosorb® M, which also are UV protection agents;
- octyldimethyl, 4-aminobenzoic acid (para-aminobenzoic acid i.e. PABA);
- octyl methoxy cinnamate (OMC) i.e. 3-(4-methoxyphenyl)-2-propenoic acid 2-ethylhexyl ester;
- benzophenon-3 i.e. 2-hydroxy-4-methoxyphenyl-phenyl methanon;
- octocrylene;
- as well as MBBT, i.e. methylene-bis-benzotriazolyl tetramethyl butyl phenol

Other known compounds used as UVA and UVB protection agents that can be applied in the compositions according to the invention are, among others, EHMC (ethylhexyl methoxycinnamate, BNBM (ethyl hexyl methoxy dibenzoyle methane), BMC (4-methyl bentzylidene camphor), BP3 (3-benzophenone), DTS (drometrizole trisiloxane), DPDT (disodium phenyl dibenzimidazole tetrasulphonate), BEMT (bis-ethyl oxyphenol methoxyphenyl triazine), IMC (isoamyl p-methoxycinnamate), PBSA (phenyl benzimidazole sulphonic acid), OT (octyl trazone), OS (octyl salicylate), TDSA (terephthalidene dicamphor sulphonic acid) and the physical and organic compounds accepted by EU and FDA as UV protectors, as well as the compounds enlisted in the INCI list, with respect to which we refer to the known literature on the field.

The effective agents contained in the composition, for example organic UV protection agents meant for a sun protection composition, are efficiently prevented from themselves turning into free radical inducing agents or even free radicals, by adding into the composition a compound mixture that inhibits the formation of said free radicals or is capable of trapping the created free radicals. When the purpose of the compound mixture is to prevent the UV protection agents from creating skin irritating free radicals, or when the purpose of the mixture is to prevent said UV protection agents from inducing the creation of free radicals in the conditions of usage of the composition, there is used a compound mixture according to the invention, by which the in vivo protective factor of the UV protection agent can be adjusted, even if its protective factor measured in vitro conditions remains the same.

In sun protection compositions manufactured according to the invention, there can also be used inorganic (mineral) UV protection agents, such as finely divided TiO₂ or ZnO, FeₓO_{y} and CeO₂, alone or together with organic UV protection agents. As was already pointed out above, these inorganic UV protection agents must be coated with a suitable protective layer, such as an aluminum oxide layer, and also dispersed in a suitable medium for preventing aggregation. In the coating of inorganic finely divided UV protection agents, there can possibly be included a mixture according to the invention, which mixture contains, as polyphenolic compounds, lignans, stilbenes, juvabions and/or flavonoids as well as their oligomers, preferably lignans and their oligomers. This kind of mixture according to the invention, extracted from a suitable raw material source, has free radical neutralizing effects, by which any undesirable effects of the free radicals created from inorganic UV protection agent are efficiently eliminated. The phenolic compounds of the mixture, including conjugated carbonyl groups, neutralize a free radical possibly formed of the inorganic protective agent (for example TiO₂) by giving up electrons to the created reactive radical, and by at the same time forming dimers. Moreover, the mixture prevents the functioning of the inorganic UV protection agent as a catalyst in aqueous conditions, where it could otherwise induce the creation of free radicals.

The compound mixture also has same properties as the UV protection agent (the mixture absorbs UV radiation and captures free radicals induced on the skin owing to the effect of UV radiation), and therefore it can also be used for boosting the protective effect against UV radiation of the inorganic UV protection agent in the composition in vivo, when the UV protective factor of the composition in vitro remains roughly the same, and/or for adjusting the protection profile of the UV protection agent in different UV ranges. By replacing part of the inorganic UV protection agent by a polyphenolic compounds containing mixture according to the invention, with economical manufacturing expenses and a good availability, the composition manufacturing expenses can be maintained the same or even reduced.

When in the composition to be produced, there is included, as the first effective agent, an organic or inorganic UV protector agent that has a certain in vitro protective factor against UV radiation, and when in said composition, there also is included a compound mixture that increases the *in vivo* protective factor against UV radiation of the composition, with respect to the *in vitro* protective factor, the compound mixture is included preferably roughly 0.3 - 0.5 wt%.

The inherent melanin of the skin, appearing a few days after exposure to UVA radiation, as stimulated by the alpha-melanocyte hormone, inhibits UV radiation from penetrating deeper in the skin layers by trapping the free radicals induced on the skin. In compositions according to the invention, there can be used UV protection agents with a UV radiation protective effect similar to that of melanin. One such UV protection agent with a melanin-like effect is a UV protection agent composition, composed of several inorganic UV protection agents and used as a semifinished product in color cosmetic compositions. This semifinished product contains extremely fine TiO₂ particles, TiO₂ pigment and iron oxides. These inorganic UV protection agents are coated, for instance as was described above, by a suitable coating layer. In this coating layer, there is included a phenolic compound mixture according to the invention, which both inhibits said inorganic UV protection agents from themselves inducing free oxygen containing radicals on the user's skin, and which mixture also has properties for protecting the skin against UV radiation.

When in the composition is included, as the first effective agent, a coated, finely divided inorganic UV protector agent, in the coating of composition is included the compound mixture, having antioxidative and/or free-radical capturing properties, so that it is capable of inhibiting the induction of free radicals from particles originating from inorganic UV protector agents. The inorganic finely divided UV protector agent is titanium oxide, iron oxide, zinc oxide or cerium oxide, the metal oxide particles of which are coated with a silica compound or with a metal oxide, such as aluminum oxide. The inorganic finely divided coated UV protector agent is dispersed in a liquid medium, such as isononyl-isononanoate, prior to its inclusion in the composition. The first effective agent to be included in the composition is preferably a UV protector agent that has an effect for protecting the skin against UV radiation, resembling the effect of natural melanin.

The free radical capture rate of polyphenolic compounds present in a compound mixture according to the invention differs from said rate in general sun protection compositions, among others, and from the rate in synthetic antioxidants BHT and BHA used as antioxidants in foodstuffs. Synthetic antioxidants, such as the above mentioned BHT and BHA, are generally cytotoxic, which means that their use as antioxidants is restricted. Moreover, the reaction speed of synthetic antioxidants also is fairly slow, and they only function in certain limited reaction conditions. On the other hand, a polyphenolic compounds containing mixture according to the invention has a remarkably low rate of cytotoxicity, when measured as the susceptibility of the mixture to create free radicals itself, and in addition to this, the free oxygenous radicals capturing capacity of the mixture per unit of time and unit of weight is different than with BHT and/or BHA. Because the mixture according to the invention has antioxidative effects, it can be used for replacing part of the share of BHT and BHA that have been found cytotoxicity, for instance in foodstuffs and sun protection compositions, or in semifinished products of sun protection compositions containing BHT or BHA. As an alternative, the mixture according to the invention can be used for ensuring a sufficient quantity of antioxidants in a composition that already contains the maximum allowed quantity of BHA or BHT, because these cannot be added in foodstuffs, owing to their cytotoxicity, more than their permitted share.

The problem with the use of both synthetic and natural antioxidants is either their slow reaction speed or their extremely selective way of functioning, i.e. they only inhibit certain oxidation reactions (synthetic antioxidants), or their thermolabile nature and their susceptibility to decomposition caused by UV light (natural antioxidants, vitamins). The reaction speed of a compound mixture according to the invention is remarkably higher than that of synthetic mixtures, and they are not especially selective but inhibit a wide range of oxidation reactions and react, among free radicals, for example with peroxide radicals, lipide radicals, superoxide-anion and peroxidase radicals. The UV light resistance of compound mixtures according to the invention is good, and they are thermally stabile.

When the first effective agent to be included in the composition is a synthetic antioxidant, in the cmposition is also included a compound mixture that in the chosen conditions of usage has a higher free-radical capture rate than said synthetic antioxidant.

The compound mixture according to the invention can also be used for technical purposes in various solvents, in liquids used in the machining of metals with chip removal, in cleaning liquids, liquid surface treatment agents, wood preparation fluids, drilling fluids etc. During usage, these liquids may get into contact with human skin, and in that case the microbicidic and bacteriostatic compounds contained therein may cause various allergic reactions and skin damages. In these technical solutions, the phenolic mixture according to the invention can be used for partly replacing for example the skin irritative microbicides contained by said solutions, or anti-micro-organistic agents; the mixture according to the invention has anti-micro-organistic effects, but it does not, however, irritate the skin as much as the synthetic anti-micro-organistic agents.

The production method of a compound mixture according to the invention, and the polyphenolic compounds contained therein, as well as the mutual ratios of their quantities depend on the designed usage and on the availability of raw materials. The mixture according to the invention is often obtained from the material of two or more wood species. Thus for example wood processing industry generally uses both spruce and pine in the pulping process. The production of a mixture according to the invention can utilize knotwood or stem knotwood parts that are less suitable in the pulping process. However, because the pulping process mainly uses spruce and to a lesser amount pine, it is often more advantageous to form a compound mixture of spruce, which is more disadvantageous for the use of the composition, because it has better availability than pine. For example, a compound mixture obtained from pine wood knot material in hydrophilic extraction has a remarkably abundant quantity of stilbenes, which are effective microbicides and effective compounds inhibiting microbial growth. As for a compound mixture obtained from spruce wood knot material in a hydrophilic extraction, it contains a remarkable quantity of 7-hydroxymatairesinol, as well as its derivatives, such as matairesinol. Although the efficiency of 7-hydroxymatairesinol in many targets of usage is lower than that of stilbenes, particularly that of pinosylvin and its derivatives, it is profitable to use in the microbicidic compound mixture knot extract obtained from spruce, containing polyphenolic compound mixture where the major components are lignans, because said extract has better availability and thus generally lower price.

The phenolic compound mixture according to the invention is produced either by pulverizing or by extracting wood material, or by combining said procedures. Because the wood material most widely available in Finland is pine or spruce, of which particularly the latter contains resin, the extraction is generally realized in two steps. Now resin compounds are extracted from the pulverized pine material, advantageously pulverized knotwood or stem knotwood material, by a lipophilic extraction solvent, and successively the polyphenols are extracted by a hydrophilic extraction solution. The lipophilic organic extraction solution is for example hydrocarbon, such as lower alkane, for example hexane or heptane. Generally the hydrophilic organic extraction solution is an organic compound containing a carbonyl group, such as alcohol or ketone. Ketone can be used only in case the compound mixture is meant for technical usage. An advantageous ketone is a lower alkyl ketone such as acetone. In case the compound mixture to be produced is a compound mixture in pulverized form, water is removed from the pulverized stemwood knot material by freeze-drying.

Advantageously the alcohol is a monovalent, bivalent or trivalent lower alkyl alcohol, or a mixture of these. The monovalent lower alkyl is preferably ethanol, propanol, butanol, heptanol, octanol or decanol. A mixture of a lower alkyl alcohol and glycerol or glycol is an advantageous solvent agent when producing several skin care compositions or semifinished products of skin care compositions. As for the lower alkylene glycol used as the hydrophilic extraction solution, it is preferably selected from a group comprising propylene glycol, butylene glycol, pentylene glycol and dipropylene glycol. Of these, the latter is particularly advantageous to be used in perfumes. In this kind of extract solution or in an extract concentrate obtained therefrom, the content of alkylene glycol is more than 7 wt%, preferably more than 10 wt%.

The composition of a compound mixture containing phenolic compounds, obtained from one and the same wood material by different extraction methods, fluctuates to some extent. The extraction method is selected according to the target of usage of the composition (for instance use in commercial solvents or cosmetic compositions), and according to the desired properties of the compound mixture. For example from Table 2 to be described below, it can be observed that the total quantities of polyphenolic lignan and stilbene compounds contained in pine knotwood and obtained by various extraction methods, as well as the mutual ratios of said polyphenolic compounds, fluctuated to some extent. Moreover, the obtained extract contained a certain amount of resin elements.

In case the extraction methods are changed, lignans can be transformed to other lignans. For example, 7-hydroxymatairesinol can in alkaline extraction conditions be transformed to alpha-conidenrin and further to alpha-conidendric acid or 7-hydroxymatairesinolic acid, and further to isohydroxymatairesinol. In acidic extraction conditions, 7-hydroxymatairesinol is transformed to isohydroxymatairesinol, and alpha-conidendric acid in turn is transformed to cyclolariciresinol, and secoisolariciresinol is in acidic conditions transformed to anhydrosecoisolariciresinol.

Hardwood species contain remarkable quantities of different flavonoids, biflavonoids and flavonoid glycosides, as is apparent from Table 3A below. Unpurified raw extracts containing flavonoids, obtained from hardwood species, have antioxidative and free radical capturing properties, wherefore they can be used for example together with raw extracts obtained from pine or spruce stem knotwood for producing compound mixtures containing phenolic compounds according to the invention, as well as their intermediate products.

It has been found out that an unpurified extract solution obtained from spruce stem knotwood material by hydrophilic extraction has, in its spectrum of influence, a similar wide-scale microbial growth-inhibiting effect as extracts obtained from pine stem knotwood (cf. Table 4B). A raw extract extracted from spruce knotwood in alcohol contains mainly lignans and oligolignans (cf. Table 3B below). Lignans contain mainly hydroxymatairesinol, secoisolariciresinol, conidendrin and oligolignans, as well as smaller amounts of other lignans such as liovile and lariciresinol. Although the microbial growth inhibiting effect of polyphenolic lignan compounds contained in a raw extract obtained from spruce stem knotwood by alcohol is weaker than with raw extracts obtained from pine stem knotwood (cf. Table 4B below), said extracts obtained from spruce stemwood chips by hydrophilic extraction can be used for producing cosmetic compositions according to the invention and their semifinished products owing to their low cytotoxic effect and low skin irritation.

In Figure 1 illustrated in Appendices 1 and 2, there are introduced polyphenolic compounds present in pine. As is apparent from Figure 1 of Appendices 1 and 2, as well as from Table 4B, pine stem knotwood and its bark contain several different stilbene compounds. Moreover, the percentual share of these stilbene compounds in the pine polyphenolic compounds present in stem knotwood and bark is remarkably high in comparison with the quantity of other polyphenolic compounds. Further, pine contains a remarkable quantity of various flavonoids.

Unpurified extracts obtained from pine stemknot wood by hydrophilic extraction contain a remarkable quantity of stilbene compounds. The applicant has verified that these extracts inhibit the growth of micro-organisms (Gram-positive and negative bacteria, fungi and yeasts) in a wide scale. Thus, pine is a good source of the anti-micro-organistic compound mixtures according to the invention. It has also been discovered that stilbenes have antiinflammatory properties. On the other hand, unpurified raw extracts obtained from spruce contain mainly lignans; the applicant has verified that said lignans have, by their spectrum of influence, a similar but weaker effect for inhibiting the growth of micro-organisms than stilbene-bearing raw extracts obtained from pine. Both raw extracts extracted from pine stem knotwood and containing mainly stilbenes, as well as raw extracts extracted from spruce stem knotwood and containing mainly lignans, are feasible when manufacturing different compositions that have an anti-microbial effect and at the same time low skin irritation and low cytotoxicity in comparison with BHT.

From the pulping processes of wood processing industry, there is obtained remarkably more spruce than pine, which fact is in favor of a solution that the compound mixtures according to the invention comprise compound mixtures of unpurified extraction solutions obtained from spruce by hydrophilic extraction, or unpurified extraction solutions obtained from spruce and pine by hydrophilic extraction. In a suitable arrangement, the combined extraction solutions according to the invention contain for example 70 wt% (poly)phenolic compounds (lignans and oligolignans) extracted from spruce stem knotwood, and 30 wt% (poly)phenolic compounds extracted from pine stem knotwood, with stilbenes included. Other mixture ratios can also be applied, as long as attention is paid to the fact that the compound mixture contained in the combined extraction solution obtained from pine and spruce has a sufficiently low cytotoxicity (the employed reference is BHT). The applicant has discovered that when the mutual mixing ratios of the raw extracts obtained from different wood species are selected so that the cytotoxicity of the compound mixture contained in the combined raw extract, when measured in ethanol for a HaCat cell culture after 24 incubations, is lower than the cytotoxicity of 0.02 -0.1 wt% BHT dissolved in ethanol in the same incubation conditions, preferably lower than the cytotoxicity of 0.01 - 0.05 wt% BHT dissolved in ethanol in the same incubation conditions, the allowed quantity for the employed mixture is 0.1 -5 wt% of the total weight of the composition, in most cases 1 - 5 wt% of the total weight of the composition.

As can be observed for example from Table 4A below, extracts obtained from the bark of different wood species by hydrophilic extraction contain various polyphenolic flavonoid compounds. In the present invention, these unpurified extracts mainly containing flavonoids can be used either as such or preferably together with hydrophilic extracts obtained from other the material of wood species, to be further used for producing various compositions according to the invention and their semifinished products. One source of these advantageous flavonoids is the bark of birch (Pendula betula). Compounds isolated from birch bark are enlisted in Table 4A and in Appendix 3 Figure 2 illustrates compounds isolated from birch bark. As can be observed from Figure 2 of Appendix 3, birch bark contains lignans, stilbenes, flavonoids, juvabiones and betuligenol and its derivatives, wherefore it can also be used as a raw material for producing an anti-micro-organistic compound mixture according to the invention. Betuligenol (betulin) mentioned at the end of Figure 2 of Appendix 3, as well as its derivatives betulinic acid, betuloinic acid or betulonic acid, can also be used in compound mixtures according to the invention, either as separately added therein in refined form, or together with an unpurified compound mixture obtained from birch bark extraction.

In Tables 5A, 5B, 5C and 5D included in Appendix, there are represented antioxidative and free radical capturing effects of pure polyphenolic compounds and of unpurified polyphenolic compounds containing raw extracts obtained from wood bark and stem knotwood.

Tables 5A and 5B represent the antioxidative effect of a few pure polyphenols and of unpurified polyphenolic compounds containing raw extracts, obtained from wood bark and stem knotwood. Tables 5C and 5D in turn illustrate the peroxide radicals capturing capacity of a few pure polyphenols and of unpurified, polyphenolic compounds containing raw extracts, obtained from wood bark and stemwood knot. In Tables 5A-5D, it can be observed that the antioxidative and free radical capturing properties of solutions containing pure polyphenol compounds, obtained from wood material in hydrophilic extraction, are often remarkably different from the corresponding properties of compound mixtures containing unpurified raw extract solutions and obtained from wood material in hydrophilic extraction, owing to the synergetic effects of the compounds contained in the compound mixtures in unpurified extracts. The compound mixtures according to the invention are obtained from these unpurified raw extract solutions.

### Production of the compositions and their semifinished products

A phenolic compounds containing mixture according to the invention can be included in cosmetic and food technology compositions and their semifinished products in a way known as such, of which examples are also given below. Thus, in case a compound mixture is extracted for instance of wood material by an alcoholic solution into a raw extract, this raw extract can be made into a homogeneous mixture such as a homogeneous solution, or a colloidal dispersion with two or several phases such as gel, paste, emulsion, microemulsion, nanoemulsion suspension, dispersion or mist. In that case a phenolic compounds containing raw extract is included in a homogeneous solution by dissolving, and/or it is included by dispersing to the carrier agents of a colloidal mixture in a way known as such, so that the raw extract is admixtured either in a phase containing a continuous carrier agent, or to a phase containing a carrier agent to be dispersed. For forming a carrier of carrier agents, there are is used conventional auxiliary agents of the trade. Such agents are, among others, surface active agents, dispersing agents such as emulsifying agents, gel formers such as carbomers and methylcellulose.

The employed carrier agents are gel base formers such as water or alcohol, cream base and paste base formers such as paraffins, waxes, silicones, aqueous phase forming agents (water) or phase formers such as paraffin or stearic acid. The compositions can also be multi-phase compositions, so that the carrier agent is formed of several aqueous and/or oil phases. With respect to the manufacturing of various compositions, we refer to the literature of this field /1/, /2/, /3/ and to the examples to be given below.

In these homogeneous solutions and colloids, there can be admixtured additives such as UV protection agents, antioxidants and vitamins, surface active agents, moisturizing agents, moisture maintaining agents, stabilizing agents, moisture absorbing agents, emollients, fats, lubricants, perfumes, viscosity regulators, colorants, antioxidants and narrow-scale anti-microbial agents etc., in a conventional way known as such, with respect to which we refer to the literature of this field /1/, /2/, /3/.

In case the composition is a packing material composition, the carrier agent is a packing material, in which the compound mixture contained in the extraction solution is impregnated or spread on. Said packing material can be cardboard, corrugated board, plastic admixtured cardboard or other packing material known from the prior art.

The first effective agent, such as a UV protection agent, antioxidants and vitamins are added in the composition in the same way as the above mentioned additives.

In case the composition is a technical composition, the carrier is a commercial solvent, cleaning device such as a cleaning cloth, solid substance such as a powder used as a surface treatment agent, or a solution meant for the cleaning of a mammal's body. In the carrier, there is impregnated or otherwise included the compound mixture according to the invention.

Surface active agents applicable in exemplary liquid compositions according to the invention, and in their semifinished compositions are: tensides, lecithin, caprylic acid and monoglycerides and diglycerides of capric acids, polyglyceryl-3-di-isostearate/polyglyceryl-2 and polyhydroxystearate, alkyl glycoside/alkyl alcohol, cetearyl pyridium chloride, bentsalkonium chloride, ionogenic agents, cetearyl glycosides, lower alcoxilated glycosides and micelle-forming agents.

Perfumes can be selected for example from a group including phenyl ethyl glycol, eugenol, isoeugenol, geraniol, citronellol or linalool, or their esterized forms or their aldehydes.

Colorants can be selected for example from among the colorants accepted by FDA to be used in foodstuffs and cosmetic products.

In compositions according to the invention, as well as in their semifinished products, it is also possible to add other effective agents as additives. Such effective agents to be used as additives are for example antioxidants. Among antioxidants, let us point out natural and synthetic vitamins such as vitamin A, B, C. D, E, provitamin B5, vitamin B3, L-ascorbic acid and vitamin E; further, there can be used antioxidants obtained from natural sources, such as antioxidants contained in green tea, antioxidants contained in flaxseed, antioxidants contained in horse chestnut, beta carotene, selenium, glutamine, ubiquinone (coenzyme Q10), glycolic acid, growth hormones and kinetin.

An advantageous botanical microbial growth inhibiting agent is betulinic acid, betuloinic acid or betulonic acid (US 6 280 778), derivatives of betuligenolin, and resvatrol obtained from spruce bark. These have been found to have an anti-microbial effect, and their cytotoxicity for healthy cells is low, and they enhance the dying of cancer cells. These can be added, either as pure compounds or as unpurified extract solutions obtained from wood bark, or as powders, in compositions and semifinished products to be manufactured according to the invention.

Further, in compositions to be manufactured according to the invention, there can also be added pure flavonoids, lignans and stilbenes as well as their oligomers isolated from plants. In this application, the term 'oligomers' refers to homologs of a compound, i.e. to its dimers, trimers etc., where the included number of similar units is lower than in a polymer. Suitable botanical polyphenol compound sources are oilseeds, nuts, grain, fruits, berries and pulses.

Several advantageous compositions to be produced by the method according to claim 1 are following:
- A sun protection composition containing: cream-like W/O or O/W carrier, as the first effective agent, an organic and/or inorganic UV protector agent, the compound mixture, emulsifier, additives such as emollient, dispersion agent, perfume and vitamins;
- A sun protection composition containing: liquid W/O or O/W carrier, as the first effective agent, a UV protector agent imitating the effect of natural melanin on the skin, where the inorganic finely divided UV protector agents are coated with a silica compound or with a metal oxide,- a compound mixture that is added in the coating of the UV protector agent,-emulsifier, additives such as viscosity regulator, moisturizer andemollient;
- A sun protection composition containing:inorganic UV protector agent, where the inorganic finely divided UV protector agents are coated with a silica compound or with a metal oxide such as aluminum oxide, a liquid, cream-like or mist-like carrier, a compound mixture that is added in the coating of the UV protector agent, emulsifier and additives;

### Examples

### Field tests

### A) Cytotoxicity in comparison with BHT

Table 1 shows comparisons between the cytotoxicity of a compound mixture according to the invention and the cytotoxicity of an antioxidant (BHT) that is widely used in food and cosmetic industry.

### Table 1

The cytotoxicity of sample extracts pulverized or alcohol-extracted of spruce stem knotwood chips, containing the compound mixture, or a powder containing the compound mixture, in comparison with the cytotoxicity of BHT (butylated hydroxytoluene). The composition of the individual compounds included in the compound mixture contained by the samples was in accordance with Table 3B. The cytotoxicity of the sample extracts and powders for human keratinocyte cells was measured. The employed measure of cytotoxicity was the total protein quantity created by the samples, when the samples were incubated together with human keratinocyte cells for a certain incubation time (24 h). For each sample, there was searched a limit value (EC20), by which 20% of the cultivated cells died.

| Extraction solvent | sample | EC20 ppm 24 h |
|---|---|---|
| pentylene glycol | HMR-5 | 450 |
| butylene glycol | HMR-4 | 600 |
| glycerol | HMR | 1.260 |
| | HMR powder | 160 |
| propylene glycol | HMR-3 | 620 |
| ethanol | HMR extract | 550 |
| ethanol | BTH | 5.50 |

From Table 1 it is observed that the most cytotoxic substance was BTH, which was 10-20 times more cytotoxic than HMR powder. HMR powder was obtained by pulverizing spruce stem knotwood without other further cleaning, and said powder contained mainly 7-hydroxymatairesinol as well as, to a lesser degree, other polyphenolic lignans. Other knot extracts obtained from spruce knotwood chips by hydrophilic extraction with alcohol (ethanol, propylene glycol, pentylene glycol, butylene glycol, or glycerol) also contained lignan mixtures according to the invention, which included, as their principal component, 7-hydroxymatairesinol and also other phenolic lignans. With respect to HMR extract mixtures, BTH was 50-100 times more cytotoxic.

### B) The growth-inhibiting effect against the micro-organisms

### Test 1

The anti-micro-organistic effect of a few compound mixtures according to the invention against bacteria, yeast and fungi was examined:
- raw extracts extracted from spruce stem knotwood chips by 4-glycol (sample 1), 5-glycol (sample 2) and 3) glycerine (sample 3) and glycerol (sample 4), containing 10 wt% of the compound mixture, the composition of the polyphenolic compounds of said raw extracts being in accordance with Table 3B (contained most 7-hydroxymatairesinol).
- combined raw extract (sample 5), extracted from spruce stem knotwood chips and pine stem knotwood by ethanol (pine) and by butylene glycol (spruce), containing roughly 10 wt% of the compound mixture obtained from pine and spruce. The sample mixture contained both a lignan mixture obtained from Norway spruce (Picea abies), the composition of which was in accordance with Table 3B, and a mixture of lignans and stilbenes obtained from pine (Pinus sylvestris), the composition of which roughly corresponded to the one illustrated in Table 2 (test 2, ethanol extraction).

The growth-inhibiting effect of the mixture samples was verified against the following micro-organisms:
Staphylococcus aureus ATCC 6538
Esterichia coli ATCC 8739
Pseudamonas auriginosa ATCC 9027
Pseudamonas putida ATCC 49128
Klebsiella pneumoniae ATCC 10031
Candida albicans ATCC 10231
Malassezia furfur ATCC 96809 (yeast fungus)
Aspergillus niger ATCC 16404

### Performance of the study

In each of the 10 g sample batches taken from the samples, there were added different microbe cell suspensions, where the microbe population density was at least 5 x 10⁶ microbes/ml. The sample batches were incubated at room temperature (22 °C) for 2, 4, 24 and 48 hours, 4 days, 7 days, 14 days and 28 days.

When reproductive microbes were not found in the samples anymore, each sample was cultivated for a 1 ml sample batch in a 100 ml Letheen broth base, and after concentration, possible bacterial growth was further checked on a culture base.

### Results

All samples had an extremely good growth-inhibiting effect against micro-organisms; after an incubation time of 4 hours, 24 hours, 48 hours, 7 days, 14 days and 28 days, microbial growth was not detected.

Both the raw extracts obtained from spruce material according to the invention by alcohol extraction, and the raw extracts obtained from pine material by alcohol extraction, as well as the raw extracts obtained from a combined spruce and pine material by alcohol extraction prevented the growth of anti-microbial agents on a wide scale. They prevented the growth of both Gram-negative bacteria E. coli, Ps. aeruginosa, Ps. putida, Kl. pneimoniae) and Gram-positive bacteria (S. aureus). In addition, they also efficiently prevent the growth of yeasts (M. furfur, C. albicans) and fungi (A. niger).

### Test 2

Raw extract extracted from spruce stem knotwood with pentylene glycol, containing a 3 wt% mixture of phenolic compounds, the composition of which was in accordance with Table 3B, was added in a sun protection cream for 0.3 wt% and 0.5 wt%, as well as known ethylhexyl glycerine (EH), efficient against Gram-positive bacteria. For the sake of comparison, to the same sun protection creams there was added a conventionally used, widely anti micro-organistic agent, phenoxyethanol (FE), which has a relatively low highest acceptable quantity of usage owing to its cytotoxicity and skin irritative properties.

| Test | number of inoculation cycles | | | | | | |
|---|---|---|---|---|---|---|---|
| | 0 | 1 | 2 | 3 | 4 | 5 | 6 |
| Test 1 | | | | | | | |
| cream base | - | - | - | +Y | +M,Y | +M,Y | ++M,Y |
| 0.3 wt% EH | | | | | | | |
| 1 wt% FE | | | | | | | |
| Test 2 | - | - | - | - | - | - | - |
| Cream base | | | | | | | |
| 0.5 wt% FE | | | | | | | |
| 1 wt% EF | | | | | | | |
| Test 3 | - | - | - | - | - | - | - |
| Cream base | | | | | | | |
| 0.3 wt% EH | | | | | | | |
| 3 wt% HMR-5 | | | | | | | |
| Test 4 | - | - | - | - | - | - | - |
| Cream base | | | | | | | |
| 0.3 5-% EH | | | | | | | |
| 3 wt% HMR-5 | | | | | | | |

| | | | | | | | |
|---|---|---|---|---|---|---|---|
| Y= yeast growth M= Fungal growth - no microbial growth + slight microbial growth ++ moderate microbial growth | | | | | | | |

As is apparent from the above described test 2, when a 3 wt% raw extract obtained from spruce stem knotwood was used in a sun protection cream instead of phenoxyethanol, it was possible to maintain a wide-scale effect against microbial growth. The effect was maintained, although the content ethylhexyl glycerine, inhibiting the growth of Gram-positive bacteria, was reduced, which shows that the raw extract from spruce stem knotwood itself has a Gram-positive bacteria growth-inhibiting effect.

The sun protection cream used in the above described test was a cream according to example 2. When using phenoxyethanol, the highest acceptable content of which is 1 wt%, there was detected a growth of yeasts and fungi with an ethylhexyl glycerine content of 0.3% in the sun protection cream. On the other hand, with an ethylhexyl glycerine content of 0.5%, the growth of yeasts and fungi was inhibited. When phenoxyethanol was replaced by a lignan mixture HMR-5 obtained from spruce stem knotwood with hydrophilic extraction (pentylene glycol), it was observed that the ethylhexyl glycerine content could be reduced to 0.3%, and yet the microbial growth in the cream was inhibited. Thus the alcohol extract (HMR-5) according to the invention, which contains a physiologically well-tolerated mixture, can be used to replace the anti-micro-organistic phenoxyethanol, the use of which is restricted owing to its physiological toxicity. The composition HMR-5 was in accordance with the composition according to Table 3B.

### C) extraction of phenolic compounds from wood material

**Table 2**

| Table 2 illustrates extracts obtained by different extraction methods from pulverized knotwood material of pine stem (scots pine). | | | | |
|---|---|---|---|---|
| | Test 1 | Test 2 | Test 3 | Test 4 |
| Compound | % of peak | % of peak | % of peak | % of peak |
| PSMME | 16 | 17 | 23 (14) | 29(16) |
| PS | 15 | 20 | 19 (12) | 20 (12) |
| NTG | 16 | 30 | 30 (18) | 33 (19) |
| Resin acids | 18 | 16 | 12 (7) | 10(6) |
| Oxidized resin acids | 35 | 17 | 17 (10) | 8(5) |

In all tests, there were extracted pine chips composed of stem knotwood. These were first extracted with hexane; in test 4, a technical hexane was used. After the extraction of lipophilic hexane, the samples were extracted with various hydrophilic solutions: in test 1 with acetone, in test 2 with ethanol (96%), in test 3 with acetone and in test 4 again with 96% ethanol. In tests 1 and 4, there were used chips which were mixture of dead and live wood material; in tests 2 and 3, the employed wood material consisted of hand-picked dead stem knotwood chips. After extraction, the quantities of the phenolic and resin compounds contained by the samples were analyzed by liquid-gas-chromatography. The contents of various compounds are given as a percentual area of the peak shown by each compound in relation to the area of all peaks. In tests 3 and 4, the weights of different compounds are given in parentheses with respect to the total weight of the solution.

Abbreviations of the compounds in the Table: PSMME: pinosylvin monomethyl ether (stilbene); PS: pinosylvin (stilbene); NTG: nortrachelogenin (lignan).

It has been discovered that raw extracts from pine knotwood are both microbial growth inhibiting on a wide scale, and also anti-inflammatory, obviously owing to the stilbene compounds contained therein, such as pinosylvin and its derivatives. Thus for example knot extracts containing unpurified phenolic compounds according to Table 2, from tests 1 and 3, can be used as such in commercial solvents. The pine knot extract obtained from examples 2 and 4, could in turn be used as such as a semifinished product for manufacturing various cosmetic compositions, without further purifying, in case the anti-micro-organistic compounds used in said products should be replaced by a mixture containing physiologically better tolerated phenolic compounds.

### D) polyphenolic compounds contained in different wood species

Mixtures according to the invention can be isolated from wood material in general. An advantageous raw material source consists of wood branches and stem's knotwood parts, but also other wood parts, such as stemwood, wood bark and needles can be used.

**Table 3A**

| The principal components of unpurified extraction solutions obtained by hydrophilic extraction from stem knotwood of various hardwood species. | | | |
|---|---|---|---|
| **Acacia crassicarpa** | | **Populus grandidentata** | |
| Flavonoids | 54% | Flavonoids | 31% |
| Melacasidine | 24% | Dihydrokaempferol | 13% |
| Isomelacasidine | 18% | Catechin | 9% |
| Biflavonoids | 9% | Naringenin | 7% |
| | | Taxifolin | 3% |
| | | Flavonoid glycosides | 34% |
| | | | |
| **Acacia mangium** | | **Populus tremula** | |
| Flavonoids | 36% | Flavonoids | 21% |
| Teracasidine | 25% | Dihydrokaempferol | 17% |
| Ketoteracasidine | 3% | Naringenin | 3% |
| Biflavonoids | 8% | Flavonoids glycosides | 8% |
| | | | |
| **Fagus sylvatica** | | **Populus tremuloides** | |
| Flavonoids | 7% | Flavonoids | 23% |
| Catechin | 6% | Dihydrokaempferol | 17% |
| | | Naringenin | 10% |
| **Eucalyptus globulus** | | Kaempferol | 1% |
| Tannins | 19% | Taxifolin | 1% |
| Tannin monemers | 5% | Flavonoid glycosides | 24% |
| Ellagic acid | 3% | | |
| Gallic acid | 2% | | |

**Table 3B**

| Polyphenolic compounds contained in a solution extracted from Norway spruce stemknot wood by pentylene glycol, as defined by a gas-liquid chromatography. The raw extract contained 87 -93 wt% pentylene glycol (solvent) and 6.5 -7.5 wt% polyphenolic compounds (mainly lignans) extracted from spruce. | |
|---|---|
| Hydroxymatairesinol | 70-80% |
| Secoisolariciresinol | 3-6% |
| Conidendrin | 4-7% |
| Lariciresinol | 1-3% |
| Liovile | 2-5% |
| Other lignans | 5-8% |

As is seen in Table 3B, spruce contains mainly lignans, of which the majority is hydroxymatairesinol. The other lignans mentioned in the Table are mainly oligolignans.

**Table 4A**

| Principal components of unpurified extraction solutions obtained from the bark of different wood species by hydrophilic extraction (Extractives in stemwood and knots of Acasia and Aspen trees, Suvi Pietarinen, Abo Akademi, Turku 2005). | |
|---|---|
| **Thuja occidentalis** | **Picea abies** |
| Sugars | Isorhapontine |
| Catechin | Astringin |
| Isorhapontine | Resveratrol-glycoside |
| Astringin | Tannins |
| Tannins | |

| **Pinus banksiana** | **Abies lasiocarpa** |
|---|---|
| Sugars | Sugars |
| Taxifolin | Resin acids |
| Isorhapontine | Tannins |
| Dihydromyrcetin | |
| Tannins | |

| **Betula pendula** | **Populus tremula** |
|---|---|
| Betuligenili glycoside | Undefined glycosides |
| Catechin | Tannins |
| Sugars | |
| Tannins | |

| **Pseudotsuga menziensii** | **Pinus mariana** |
|---|---|
| Sugars | Sugars |
| Taxifolin | Catechin |
| Catechin | Tannins |
| Tannins | |

**Table 4B**

| Principal components of unpurified extracts obtained from the stem knotwood of a few pine and spruce species by hydrophilic extraction (Wilför et al., J. Agric. Food. Chem., 51, 26 (2003)), in percentages by weight of the total quantity of the components of the mixture extracted from the wood. | | |
|---|---|---|
| **Wood material** | **Compounds** | **wt%** |
| Picea abies | | |
| | **Lignans** | 53 |
| hydroxymatairesinol | | 41 |
| secoisolariciresinol | | 3 |
| α-conidendrin | | 7 |
| Oligolignans | | 12 |
| | | |
| Abies sibirica | | |
| **Lignans** | | 33 |
| secoisolariciresinol | | 21 |
| lariciresinol | | 7 |
| Oligolignans | | 31 |
| **Juvabiones** | | 3 |
| | | |
| Abies balsamea | **Lignans** | 22 |
| secoisolariciresinol | | 18 |
| lariciresinol | | 9 |
| | **Oligolignans** | 19 |
| | **Juvabiones** | 2 |
| | | |
| Pinus sibirica | | |
| | **Lignans** | 26 |
| lariciresinol | | 19 |
| isolariciresinol | | 3 |
| secolariciresinol | | 2 |
| Oligolignans | | 6 |
| | **Flavonoids** | 7 |
| pinosembrin | | 6 |
| | **Stilbenes** | 25 |
| dihydropinosylvin monomethyl ether | | 15 |
| pinosylvin | 3 | |
| dihydropinosylvin | | 2 |
| | | |
| Pinus contorta | | |
| | **Lignans** | 10 |
| nortrachelogenin | | 5 |
| liovile | | 3 |
| oligomers | 3 | |
| | **Flavonoids** | 20 |
| pinosembrin | | 15 |
| pinobanxin | 7 | |
| | **Stilbenes** | 15 |
| Pinosylvin monomethyl ether | | 9 |
| Pinosylvin | 6 | |

Polyphenolic and phenolic compounds isolated from birch bark are illustrated in Figure 2 of Appendix 3. As can be observed from Figure 2, also birch bark contains lignans, stilbenes, flavonoids and juvabiones as polyphenolic compounds, wherefore it can also be used as a raw material for producing a compound mixture according to the invention. Phenolic betuligenol, mentioned at the end of Figure 2, can also be used in compound mixtures according to the invention, either as separately added therein in separately purifiedform, or together with an unpurified compound mixture obtained from birch bark extraction.

### E) Increasing the in vivo-protective factor of sun protection composition

For a sun protection cream according to example 4, in which there was mixed raw extract for 3 wt%, said raw extract being obtained by extracting chips from stem knotwood of spruce by pentylene glycol, there was tested the protective factor (SPF) both in vitro and in vivo.

The protective factor in vivo was defined on the skin of 5 test persons according to the method "International SPF test method 2006" (International SPF test method 2006, CTFA South Africa, JCIA and CTFA, 2006). The radiation source used for UV irradiation was "Multiport Solar UV Simulator Type 601 -150". The *in vivo* protective factor obtained for the sun protection composition by this method was 61.0.

For a similar sun protection composition, the obtained *in vitro* protective factor, by the "Diffrey-Robson" standard method, was 40.

### Production of the compositions and their intermediate products

### Example 1

Intermediate product: semifinished product with a UV protection profile similar to that of melanin.

Pigment-quality TiO₂ and extremely finely divided TiO₂, as well as pigment-quality iron oxide is coated for example with aluminum oxide and with a mixture of polyphenolic compounds according to the invention, which mixture has antioxidative and/or free radical capturing properties. The mineral UV protection agents (TiO₂ and iron oxide) to be coated are dispersed to an oil phase, such as isononyl-isononanoate, by means of a surface active compound. A suitable mixture of polyphenolic compounds is for example a lignan mixture extracted from spruce knotwood material by butylene glycol or pentylene glycol, which contains 7-hydroxymatairesinol as its principal component, and where the polyphenolic compounds contained by the mixture are similar to those represented in Table 3B, so that the relative quantities of the polyphenolic compounds contained in the mixture are mutually similar to those represented in Table 3B.

### Example 2

Semifinished product: inorganic semifinished product containing UV protection agent.

The semifinished product contains inorganic and/or organic UV protection agents, such as TiO₂ or TiO₂ and ZnO, as well as possibly organic MBBT absorbing radiation within the UVA and UVB ranges. Inorganic UV protection agents are coated with aluminum oxide or silica, and in the coating, there is included a mixture according to the invention, containing polyphenolic compounds. Here a suitable mixture is for example a solution extracted from knotwood material of picea abies, first by hexane and then by butylene glycol, which contains 7-hydroxymatairesinol and other lignans. As the principal component, the raw extract contained 7-hydroxymatairesinol and also lesser but still detectable quantities of lariciresinol, conidendrin and oligolignans. Inorganic UV protection agents are coated in an oil dispersion, where the continuous phase is for example a dimeticon or isononyl-isononanoate, in the presence of a suitable surface active agent.

In the semifinished products of both Example 1 and Example 2, the inorganic UV protection agent is well protected by a mixture of polyphenolic compounds according to the invention, and it can neither induce the creation of free radicals nor be itself transformed into a free radical. The polyphenolic mixture present in the coating of the UV protection agent is itself stabile, and it also has UV radiation absorbing properties, which means that it can be used for modifying the UV radiation absorption profile of the compositions within the UVA and UVB ranges.

### Example 3

### UVA/UVB Sun Protection Lotion, O/W type, with TINOSORB® M

Lotion with a very high SPF and excellent UVA protection due to the photostable UVA filter TINOSORB® M. This emulsion is smooth and spreads easily. SPF in vivo = 38, broadband.

This lotion includes UVA filter TINOSORB® M and also 10 wt-% Wood Knot Extract in Glycerine, which will enhance in vivo SPF by trapping possible free radicals including reactive oxygen and lipid radicals induced by solar ultraviolet radiation or particles from organic UV filters.

| **Composition** | | | | |
|---|---|---|---|---|
| | **Trade name** | **Inci Name** | **Supplier** | % w/w (as supplied) |
| Part A | Amphiosol K | Potassium Cetyl Phosphate | Roche | 2.00 |
| | Antaron WP-660 | Tricontanyl PVP | ISP | 1.00 |
| | Myritol 318 | Caprylic/Capric Triglyceride | Cognis | 5.00 |
| | Crodamol AB | C12-15 Alkyl Benzoate | Croda | 5.00 |
| | Cetiol SN | Cetearyl Isononanoate | Cognis | 5.00 |
| | Cutina GMS | Glyceryl Stearate | Cognis | 3.00 |
| | Lanette 16 | Cetyl Alcohol | Cognis | 1.00 |
| | Dow Corning 200 Fluid 350 cs | Dimethicone | Dow Coming | 0.10 |
| | **TINOSORB™ OMC** | Ethylhexyl Methoxycinnamate | Ciba Specialty Chemicals | 5.00 |
| | | | | |
| Part B | Water | Water | | q.s. to 100 |
| | | 10 wt% Wood Knot Extract in Glycerine | Granula Ltd | 3.00 |
| Part C | **SALCARE**® **SC80** | Steareth-10 Allyl Ether/Acrylates Copolymer | Ciba Specialty Chemicals | 0.50 |
| | | | | |
| Part D | **TINOSORB**® **M** | Methylene Bis-Benzotriazolyl Tetramethylbutylphenol (and) Aqua (and) Decyl Glucoside (and) Propylene Glycol (and) Xanthan Gum | Ciba Specialty Chemicals | 20.00 |
| | | | | |
| Part E | Phenonip | Phenoxyethanol (and) Methylparaben (and) Ethylparaben (and) Butylparaben (and) Propylparaben (and) Isobutylparaben | Clariant | 0.30 |
| | | | | |
| Part F | Sodium Hydroxide (Solution 10%) | Water (and) Sodium Hydroxide | | q.s. to pH 7.00 |
| | | | | |
| Part G | Fragrance | Fragrance | | q.s. |

| ***Technical Data*** | |
|---|---|
| pH value | 7.00 |
| Appearance | white lotion |
| Viscosity (Brookfield DVIII+LV4/80rpm) | 3000 mPas |
| UVA/UVB ratio* / Critical Wavelength* | 0.75 / 384 nm |

### Example 4

### Sun protection cream

In this sun protection cream, in order to obtain a wide-scale anti-microbial effect, there was used a compound mixture (HMR-5) extracted from stem knotwood chips from picea abies in pentylene glycol. HMR-5 was an unpurified raw extract and contained 10 wt% of the compound mixture and 90 wt% of pentylene glycol. At the same time, HMR-5 also served as a trapper of free radicals induced by possible sunlight or by particles released from an organic UV protection agent, thus increasing the in vivo protective factor on the skin. The sun protection cream contained UV protection agents that protect the skin both against UVA and UVB rays (Granlux GA12-45 and an organic protection agent).

| Part 1 | |
|---|---|
| Granlux GA12-45 : | 28% |
| Concentrate containing inorganic UVprotection agents (TiO₂ and ZnO) protected by aluminum oxide, dispersed in dimethicone and surface active dispersing agent | |
| Glycerine | 4% |

| Part 2 | |
|---|---|
| alpha-Bisapolol | 1.5% |
| Bytospermum parkii Fruit | 1.5% |
| Cetiol CC | 0.1% |
| Oxynex K Fluido | 0.1% |
| Alkyl benzoate | 4% |

| Part 3 | |
|---|---|
| Water | 23.5% |
| Aloe barnadensis | 1% |
| Multivitamin product | 0.3% |
| Dinatrium EDTA | 0.1% |

| Part 4 | |
|---|---|
| Cyclomethicone | 1.5% |
| Ethylhexyl methoxycinnamate | 5% |
| Organic UV protection agent | 2% |
| Homosalate | 5% |

| Part 5 | |
|---|---|
| Ethylhexyl glycerine | 0.5% |
| HMR-5 (intermediate product of example 1) | 3% |

Parts 1-5 were mixed as a sun protection cream by conventional methods.

### Example 5

### Cleaning cloth containing anti-microbial compound mixture according to the invention

This can be manufactured as is described in the US patent 6 287 582. The cloth contains a water-insoluble carrier and a cosmetic composition impregnated in the carrier, including a compound mixture according to the invention, pH regulator such as alpha- or beta-hydroxycarboxylic acid, silicone microemulsion and surface active agent. The pH of the cosmetic composition in water is no more than 6. The microemulsion is for example a dimethiconol microemulsion.

In the Table below, there are given two exemplary compositions to be impregnated in a cleaning cloth, containing an extract mixture according to the invention.

| Ingredient | Acceptable range (Percent by weight) |
|---|---|
| Dual chain quaternary (N-alkyl dimethyl Ethylbenzyl chloride or N-alkyldiemethyl Ethylbenzyl ammonium chloride) | 0.0 - 2.0 |
| Ortho phenyl phenol | 0.0025 - 2.0 |
| Paratertiary amyl phenol | 0.0025 - 2.0 |
| Extracted mixture in alcohol (Granula Ltd) | 1.0 - 40.0 |
| Tergitol 15-S-5 | 0.5 - 2.0 |
| Citric acid | 0.1 - 2.0 |
| Emollient | 0.1 - 3.0 |
| Water | Up to 100 |
| Ingredient | Specific composition formulation (Percent by weight) |

| | |
|---|---|
| Dual chain quaternary (N-alkyldimethylethylbenzyl chloride or N-alkyldimethylethylbenzyl ammonium chloride) | 0-0.25 |
| Ortho phenyl phenol | 0.0125 |
| Paratertiary amyl phenol | 0.0025 |
| Extracted mixture in alcohol (Granula Ltd) | 0.01-40.0 |
| Tergitol 15-S-5 | 0.5 |
| Citric acid | 0.05 |
| *Aloe vera* gel | 1.0 |
| Water | Up to 100 |

### Example 6

### Aftershave gel without alcohol

| **Trade name/INCI** | **p-%** |
|---|---|
| **A** | |
| Carbopol 940 /Carbomer | 0.30 |
| Water dem. Aqua dem. | 40.00 |
| | |

| **B** | |
|---|---|
| Cremophor CO 40 / PEG-40/ Hydrogenated castor oil | 3.00 |
| | |
| Perfume | q.s. |
| Menthol | 0.10 |
| D-Panthenol 50 P/Panthenol, | 0.10 |
| 10% extracted mixture in propylene glycol | 4.00* |
| Triethanolamine | 0.40 |
| Water Aqua dem. | up to 100% |

### Production

Let phase A swell. Phase B is dissolved and mixed in phase A.
Viscosity: roughly 4 000 mPa s (Brookfield RVT), pH value roughly 7.
*a mixture of phenolic compounds obtained from stem knotwood chips in propylene glycol extraction, contg. 10 wt% of the compound mixture and 90 wt% of propylene glycol.

### Example 7

### Rehydrating aftersun mist

In the production of this aqueous mist-like composition, there was used a water component, emulgator (Luviquat®Mono CP) and PEG-40, a surface active silicone-based agent (Dow Coming 190 Surfactant), a moisturizer (D-panthenol), and a moisturizing agent Prodew® 200. The growth of micro-organisms was inhibited by a mixture of phenolic compounds, obtained in pentylene glycol extraction from chips contained in spruce stem knotwood in mixture, contg. 10 wt% of the compound mixture and 90 wt% of propylene glycol. This raw extract was used unpurified in the production of mist.

| **Trade name/INCI** | **wt%** |
|---|---|
| **A** | |
| Deionized water | 89.10 |
| Luviquat®Mono CP /Hydroxyethyl cetyl dimonium phosphate (1) | 2.00 |
| D-Panthenol (1) | 0.50 |
| 10% extracted mixture in pentylene glycol (5)*' | 5.50 |
| Dow Corning 190 Surfactant /Dimethicone polyol (2) Prodew® 200 /Sodium lactate & Sodium PCA & Sorbitol & | 0.50 |
| Hydrolyzed Collagen & Proline (3) | 2.00 |
| | |
| **B** | |
| Cremophor® RH 40 /PEG-40, Hydrated castor oil (1) | 0.30 |
| Perfume | 0.10 |

| | |
|---|---|
| * polyphenolic mixture obtained from wood chips contained in stem knotwood in pentylene glycol extraction, contg. 10 wt% of the extract mixture and 90 wt% of propylene glycol. | |

### Production

Combine ingredients of phase A and stir until solution is clear.

Combine ingredients of phase B. Melt hydrated castor oil and stir with perfume. Combine phase B with A and stir until mixture is clear.
pH of the end product is 6.

### Suppliers

1) BASF (2) Dow Coming (3) Ajinomoto (4) Nipa (5) Granula Ltd

### Example 8

### Body milk

The body milk according to Example 6 is an aqueous emulsion, in the manufacturing of which there are used emulsifying agents (Cremophor), auxiliary agent (glyceryl monostearate) as well as water and oil components, viscosity regulator (cetyl stearyl alcohol), emollients (Luvitol EHO) and conditioner (Luviquat PQ 11). The growth of micro-organisms was inhibited by a mixture of phenolic compounds obtained from spruce stem knotwood in butylene glycol extraction, which mixture contained phenolic compounds 10 wt%, and which raw extract was used unpurified for the production of body milk.

| **Trade name/ INCI** | **wt %** |
|---|---|
| **A** | |
| Cremophor A 6/ Ceteareth-6, Stearyl alcohol | 1.00 |
| Cremophor A 25/ Ceteareth-25 | 1.00 |
| Glyceryl monostearate | 2.00 |
| Cetyl stearyl alcohol | 2.00 |
| Paraffin oil/ Mineral oil | 3.00 |
| | |
| Luvitol EHO/ Cetyl stearyl octanoate | 5.00 |
| **B** | |
| 10 wt% extracted mixture in butylene glycol* | 5.00 |
| Luviquat PQ 11 (1) Polyquaternium-11 | 4.00 |
| Water | 77.00 |
| | |
| **C** | |
| Perfume | q.s. |

| | |
|---|---|
| ** polyphenolic mixture obtained from wood chips contained in stem knotwood in butylene glycol extraction, contg. 10 wt% extract mixture and 90 wt% butylene glycol. | |

### Production

Mix phases A and B separately at roughly 80 °C. Mix phase B to phase A whilst homogenizing, and continue homogenizing for a while. Cool roughly at 80 °C, add phase C and homogenize again.
Viscosity: roughly 3000mPas
pH: roughly 6

### Example 9

### Concentrated powder

| **Trade name/ INCI** | **wt %** |
|---|---|
| **A** | |
| Talcum | 72.00 |
| Magnesium stearate | 10.00 |
| Calcium carbonate | 2.00 |
| Sicovit White E 171/C,I. 77891/Titanium oxide | 9.00 |
| Sicovit Brown 70 E 172/Ferric oxides | 1.00 |
| Powdered mixture | 5.00 |
| | |

| **B** | |
|---|---|
| Paraffin oil/ Mineral oil | 0.50 |
| Vaseline/ Petrolatum | 0.50 |

### Production

Mix ingredients of phase A and homogenize. Stir phase B to phase A and mix again.

### Example 10

### Cell-protective composition

| **Trade name, compound /INCI** | | **wt%** |
|---|---|---|
| **A** | | |
| RonaCare™ Ectoin (1) | | 1.00 |
| 10% extracted mixture in pentylene glycol (5) | | 3.00 |
| Water, mineralized | | up to 100% |
| | | |

| **B** | | |
|---|---|---|
| Sisterna SP30-C (2) / | saccharose distearate | 2.70 |
| Sisterna SP70-C (2) / | saccharose stearate | 0.90 |
| Cetiol OE | (3) / dicapryl ether | 5.00 |
| Miglyol 812 | (1) / kaprylic/kapiic triglyceride | 2.00 |
| Isopropyl palmitate | (3) / isopropyl palmitate | 2.00 |
| Cegesoft C 24 | (3) / ethylhexyl palmitate | 7.00 |
| Carbopol ETD 2001 | (4) / carbomer | 0.20 |
| | | |

| **C** | | |
|---|---|---|
| Sodium hydroxide, 10% solution (1) /sodium hydroxide q.s. | | |

### Production

Heat phase A to75° C, disperse phase B and heat to 75° C, add phase B to phase A, homogenize, adjust pH with sodium hydroxide, cool to room temperature by stirring simultaneously.

### Note

pH (22° C): 6.50
Viscosity (21° C): 109 000 mPa.s (Brookfield RVT, spindle C, 5 rpm, Helipath)

### Suppliers

(1) Merck KGaA/Rona®
(2) Sisterna C.V./ Dai-Ichi
(3) Cognis GmbH
(4) BF Goodrich GmbH
(5) Granula Ltd

### Example 11

### Night care cream

For producing this cream-like composition, there was used a water component, emulsifying agents (PEG-7), emollients (Luvitol EHO), wax components and fungicidal and moisturizing agents (jojoba oil). The growth of anti-microbial agents was prevented by jojoba oil and by a mixture of phenolic compounds, obtained from spruce stem knotwood in glycerine extraction, which mixture contained phenolic compounds 10 wt%, and which raw extract was used unpurified for producing the cream.

| **Trade name/ INCI** | **wt %** |
|---|---|
| **A** | |
| Cremophor WO 7 / PEG-7 Hydrated castor oil | 6.00 |
| Luvitol EHO /Cetearyl octanoate | 5.00 |
| Permulgin 3220 /Microcrystalline wax | 2.00 |
| Beeswax | 0.50 |
| Cetiol SB 45 | |
| /Shea Butter (Butyrospermum parkii) | 0.50 |
| Jojoba oil/Jojoba (Buxus chinensis) oil | 2.00 |
| Paraffin oil/Mineral oil | 10.00 |
| | |

| **B** | |
|---|---|
| 10% extracted mixture in glycerin | 5.00 |
| Water | 67.00 |
| | |

| **C** | |
|---|---|
| Sodium ascorbyl sorbate | 2.00 |
| Perfume | q.s. |

### Production

Mix phases A and B separately to roughly 80 °C. Stir phase B to phase A whilst homogenizing, continue homogenizing for a while. Cool to roughly 40 °C, add C and homogenize again.

### Example 12

### Night care cream

The night care cream according to example 10 was almost identical to night care cream of example 9, but instead of sodium ascorbyl sorbate of example 9, sodium ascorbyl phosphate was used in example 10 as an antioxidant agent.

| **Trade name/ INCI** | **wt %** |
|---|---|
| **A** | |
| Cremophor WO 7 / PEG-7 Hydrogenated Castor Oil 6.00 | |
| Luvitol EHO / Cetearyl Octanoate | 5.00 |
| Permulgin 3220 / Microcrystalline Wax | 2.00 |
| Beeswax | 0.50 |
| Cetiol SB 45 / Shea Butter (Butyrospermum parkii) | 0.50 |
| Jojoba Oil/Jojoba (Buxus chinensis) Oil | 2.00 |
| Paraffin Oil/Mineral Oil | 10.00 |
| | |

| **B** | |
|---|---|
| 10% extracted mixture in glycerin | 5.00 |
| Water | 67.00 |
| | |

| C | |
|---|---|
| Sodium Ascorbyl Phosphate | 2.00 |
| Perfume | q.s. |

### Production

Heat phases A and B separately to about 80° C. Stir phase B into phase A whilst homogenizing and continue homogenizing for a while. Cool to about 40° C, add phase C and homogenize again.

### Example 13

### Softcream with vitamin E

For making softcream of example 13, there were used several emollients, oil and water components, preservatives, adjuvants and other additives including vitamins for antioxidant purpose. Antioxidative properties of vitamins was enhanced by adding a mixture of 10 wt% raw-extract (from spruce) including phenolic compounds in Glycerin (10 wt% of mixture of compounds and 90 wt% of glycerin). This glycerin containing raw-extract was used without further purification. The reactions of vitamins are generally slow and therefore they are not able to function in every environment sufficiently.

| **Trade name/ INCI** | **wt%** |
|---|---|
| **A** | |
| Cremophor GO 32 / Polyglyceryl-3 Dioleate | 0.75 |
| Luvitol EHO / Cetearyl Octanoate | 7.50 |
| Finsolv TN / Alkyl Benzoate | 5.00 |
| Miglyol 812 / Caprylic/Capric Triglyceride | 4.00 |
| Abil EM 90 / Cetyl Diethicone Copolyol | 2.25 |
| Abil 350 / Dimethicone | 1.50 |
| Ascorbyl Palmitate, Citric Acid, Glyceryl Stearate, Propylene Glycol | 0.20 |
| | |

| **B** | |
|---|---|
| 10% extracted mixture in Glycerin | 0.75 |
| Sodium Hydroxide | 0.25 |
| D-Panthenol USP/Panthenol | 1.50 |
| Sodium Chloride | 1.50 |
| EDTA | 0.1 |
| Preservative | q.s. |
| Water | 69.80 |
| | |

| **C** | |
|---|---|
| (-)-Alpha-Bisabolol nat./Bisabolol | 0.10 |
| Vitamin A Palmitate 1 Mio./Retinyl Palmitate | 0.10 |
| Vitamin E Acetate/Tocopheryl Acetate | 5.00 |
| Perfume | q.s. |

### Production

Heat phases A and B separately to about 80° C. Stir phase B into phase A whilst homogenizing. Cool to about 40° C, add phase C and homogenize again.
Viscosity: approx. 18 000 mPas

### Example 14

### Multi-vitamin cream, TYP W/O Formula

For manufacturing this W/O-type cream composition water and oil were used as carrier agents, as an adjuvant emulgators (PEG-7, PEG-45, Claytone XL) and as an additive moisturizer (Jojoba oil,) perfume and vitamins (sodium ascorbyl phosphate and retinol). Antioxidative and radical trapping capacity of vitamins was modified by a mixture of 10 wt% extracted mixture (spruce) containing phenolic compounds extracted from spruce knots into butylene glycol (10 wt % of mixture of phenolic compounds and 90 wt% of butylene glycol). This raw-extract was used without further purification. Vitamins and extracted mixture of compounds have a different time scale during which they will trap free radicals and therefore they complete each other.

| **Trade name/INCI** | **wt%** |
|---|---|
| **A** | |
| Cremophor WO 7 /PEG-7 Hydrogenated Castor Oil | 6.00 |
| Paraffin Oil / Mineral Oil | 10.00 |
| Vaseline/ Petrolatum | 3.00 |
| Miglyol 812/ Caprylic/Capric Triglyceride | 5.00 |
| Elfacos ST 9/PEG-45/Dodecyl Glycol Copolymer | 2.00 |
| Jojoba Oil/ Jojoba (Buxus Chinensis) Oil | 5.00 |
| Claytone XL/ Quaternium-18 Bentonite | 1.00 |
| | |

| **B** | |
|---|---|
| 10% Wood Knot Extract in Butylene Glycol | 4.00 |
| EDTA | 0.10 |
| Water | 61.90 |
| | |

| **C** | |
|---|---|
| Sodium Ascorbyl Phosphate | 1.00 |
| Retinol | 1.00 |
| Perfume | q.s. |

### Production

Heat phases A and B separately to about 80° C. Stir phase B into phase A whilst homogenizing and continue homogenizing for a while. Cool to about 40° C, add phase C and homogenize again. Viscosity: approx. 14 000 mPa s (Haake Viscotester VT-02).

### Example 15

### GAI-45 TS High SPF cream

This composition was made of water phase, semifinished composition of GranLux® GAI-45 TS which is an W/O emulsion including UV protective agent (TiO₂ filter) in a silicon emulsifier system and 10 wt% mixture of phenolic compounds originating to spruce knots and extracted into pentylene glycol (10 wt% of mixture of phenolic lignan compounds and 90 wt% of pentylene glycol). The extracted mixture will protect the coating of TiO₂ filter preventing particles of UV filter to induce free radicals at skin. The end composition included also emollient and dispersive oil (isononyl-isononanoate) and perfume.

| **Trade name/INCI** | **Amount (%)** | **Manufacturer** |
|---|---|---|
| **A** | | |
| GranLux® GAI-45 TS | 25.0 | Granula Ltd |
| 10% extracted mixture | | |
| in pentylene Glycol | 3.0 | Granula Ltd |
| | | |

| **B** | | |
|---|---|---|
| Water | 10.0 | |
| | | |

| C | | |
|---|---|---|
| Isononyl Isononanoate | 22.0 | Seppic |
| | | |

| **D** | | |
|---|---|---|
| Water | 39.0 | |
| Perfume | q.s. | |

1) Mix A at room temperature.
2) Prepare B and add it to A. Mix ca 3-5 min until all water has been taken up. The water will go in by diffusion, hydrate the polar parts and form the liquid crystalline phase. The polar phase and the hydrophobic phase seem initially to be totally separated but the water phase will be taken up by time and mixing.
3) Add to C to A+B while mixing. Viscosity goes down.
4) Add D to C+A+B slowly (during ca 5 minutes) while processing well (Ystral speed 3-5) for totally 15 minutes.
SPF : well over 30 (SPF in vitro 49 +/- 3)
UVA: fulfills "Australian Standard"

### Example 16

### Fluid foundation using Granlux™ Melanin

### Mimic™ TB concentrate

For making this composition there was used a semi-composition Granlux™ Melanin Mimic™ which is a formula imitating the UV protection of natural melanin. Growth of micro-organisms (bacteria, fungi, yeast) was inhibited by a mixture of 10 wt% mixture of phenolic compounds extracted from spruce knots in pentylene glycol (10 wt% of mixture of phenolic compounds and 90 wt% of pentylene glycol ). This raw extract was used without further purification. Also betulonic acid originating to birch bark was used as an anti-microbial agent.

| **Trade name/INCI** | **Amount (wt%)** | **Manufacturer** |
|---|---|---|
| **A** | | |
| Magnesium aluminum silicate | 0.70 | |
| (Veegum K) | | Vanderbilt |
| Xanthan gum | 0.30 | |
| (Rodicare) | | Rhone Poulenc |
| 10% extracted polyphenolic mixture in pentylene glycol | 6.00 | Granula Ltd |
| Glycerine | 4.00 | |
| Deionized water | q.s. | |

Wet the Xanthan gum in water + glycerine + 10wt% polyphenolic mixture extracted from spruce knots into pentylene + 10wt% Betulonic acid in propylene glycol. Homogenize with turboemulsifier and add Magnesium aluminum silicate while mixing, heat to 75° C.

| **B** | | |
|---|---|---|
| Granlux™Melanin Mimic™ | 27.50 | Granula Ltd |
| Limnanthes alba; | | |
| Butyrospermum parkii | 3.50 | The Fanning Co |
| (Fancol VB) | | |
| Glyceryl stearate | 0.80 | Th. Goldschmidt |
| (Tegin M) | | |
| Isopropyl myristate | 4.00 | |
| Isohexadecane | 10.00 | ICI |
| (Arlamol HD) | | |
| Stearic acid | 2.00 | |
| Dimethicone | 1.00 | Dow Coming |
| (Dow Coming 200 Fluid 100 cs) | | |

Melt Phase B at 65° C, slowly homogenizing for ca 5 min, heat to 75° C.

| **B1** | |
|---|---|
| Talcum | 1.00 |

Add Phase A to Phases B while homogenizing. As emulsion is formed, add Phases B1 and C slowly while continuing homogenizing.

| **C** | | |
|---|---|---|
| Triethanolamine | 1.50 | |
| | | |

| **D** | | |
|---|---|---|
| PPG 25 Laureth 25 | 0.20 | Vevy |
| (ADF Oleile) | | |

At 40° C add Phase D while homogenizing. Cool to room temperature while mixing.

Characteristics:
pH: ca 7
Viscosity: 6.000
SPF: 21-24

### Example 17

### Soft colored cream (SCC/EM/98)

| **INCI(Trade name)** | **Amount (%)** | **Manufacturer** |
|---|---|---|
| **A** | | |
| Magnesium aluminum silicate | 0.50 | Vanderbilt |
| (Veegum K) | | |
| Xanthan gum | 0.50 | |
| (Rodicare) | | Rhone Poulenc |
| Propylene glycol | 6.00 | |
| 10% Betulonic acid in Glycerine | 4.00 | Granula Ltd |
| Deionized water | | up to 100% |

Wet the Xanthan gum in water + betulonic acid in glycerine + propylene glycol. Homogenize with turboemulsifier and add Magnesium aluminum silicate while mixing, heat to 75° C.

| **B** | | |
|---|---|---|
| Granlux™ EM-50 | 10.00 | Granula Ltd |
| Limnanthes alba; | | |
| Butyrospermum parkii | 3.50 | The Fanning Co |
| (Fancol VB) | | |
| Glyceryl stearate | 0.80 | Th. Goldschmidt |
| (Tegin M) | | |
| Isopropyl myristate | 4.00 | |
| Isohexadecane | 10.00 | ICI |
| (Arlamol HD) | | |
| Polydecene | 4.00 | Fortum |
| (Nexbase 2004 FG) | | |
| Polyhydroxystearic acid | 0.50 | ICI |
| (Arlacel P100) | | |

Melt Phase B at 65° C, add Phase B1 slowly homogenizing for ca 5 min, heat to 75° C.

| **B1** | | |
|---|---|---|
| Cl 77492 | 1.40 | |
| (Ariabel yellow) | | Warner&Jenkinson |
| Cl 77491 + Cl 77492 | 0.30 | |
| (Ariabel sienna) | | Warner&Jenkinson |
| Cl 77491 + Cl 77492 Cl 77499 | 0.30 | |
| (Ariabel umber) | | Warner&Jenkinson |
| Cl 77891 (Titanium dioxide) | 6.00 | |
| (Kemira AFDC) | | Kemira |

Add Phase A to Phases B + B1 while homogenizing. As emulsion is formed, add Phase C while continuing homogenizing.

| **C** | |
|---|---|
| Talcum 1.00 | |
| Aluminum starch octenylsuccinate | 3.00 |
| (Dry-Flo PC) | National Starch |

| **D** | | |
|---|---|---|
| PPG 25 Laureth 25 | 0.20 | |
| (ADF Oleile) | | Vevy |
| Propylene glycol; Diazolidinyl urea; | | |
| Methyl paraben; Propylparaben | 1.00 | |
| (Germaben II E) | | ISP |

At 40 °C add Phase D whilst homogenizing. Cool to room temperature while mixing. Note: During the preparation the phase inversion temperature is clearly noticeable (PIT ca 40°) since the W/O system previously formed breaks into two phases: one liquid and one creamy. While continuing homogenization, the final emulsion (O/W) is easily obtained. The low value of PIT is not related to unstable behavior, in fact the formulation is still stable after 4 months at 42° C.

Characteristics:
pH: ca 7
Viscosity: 180.000 mPa s RVT Brookfield (5 rpm, 298 K, Helipath Stand T-D
SPF: 21-23 in vitro, UVA/UVB = 0.77

### Example 18

A stick with UV protection of SPF 15 was made from semi-composition of Granlux CCA-50, which includes mainly physical filter for UV protection, beeswax and carnauba wax. Large-scale protection against micro-organisms (bacteria, yeast, fungi) is achieved by a powdered compound mixture originating to pulverized spruce knots.

### SPF 15 Stick

| | | |
|---|---|---|
| Hydrogenated Vegetable Oil (Cremeol HF-52) | 15.0 | Aarhus Olie |
| Vegetable Oil (Cremeol PS-6) | 68.0 | Aarhus Olie |
| Candelilla wax | 6.0 | |
| Powdered mixture | 1.0 | Oy Granula Ab, Ltd |
| Granlux CCA-50 | 10.0 | Oy Granula Ab, Ltd |

Heat ingredients to 75-80 °C. Mix until uniform. Cool to 50 °C. Pour into moulds. Characteristics:
SPF: 13-15 in vitro
UVA/UVB ratio 0.56

### Example 19

A stick with UV protection of SPF 30 was made from semi-composition of Granlux CCA-50, which includes mainly physical filter for UV protection, beeswax and carnauba wax. Large-scale protection against micro-organisms (bacteria, yeast, fungi) is achieved by a powdered compound mixture originating to pulverized spruce knots.

### Formula: SPF 30 Stick

| **Trade name/ INCI** | **wt %** | |
|---|---|---|
| Beeswax (Cera alba) | 12.0 | |
| Caprylic Capric Triglycerides | 12.5 | |
| Macadamia nut oil | 9.5 | |
| Cetearyl alcohol | 7.5 | Henkel |
| Petrolatum | 36.5 | |
| Granlux CCA-50 | 20.0 | Oy Granula Ab, Ltd |
| Powdered mixture | 2.0 | Oy Granula Ab, Ltd |

Heat ingredients to 75-80 °C. Mix until uniform. Pour into molds.
Characteristics:
SPF: 28-30 in vitro

### Example 20

### Sun protection gel

| **Trade name/ INCI** | **wt %** |
|---|---|
| **A** | |
| Uvinul MC 80/Octyl Methoxycinnamate | 8.00 |
| Uvinul N 539 T/Octocrylene | 5.00 |
| Uvinul M 40 /Benzophenone-3 | 2.00 |
| Parsol 1789/Butyl Methoxydibenzoylmethane | 0.80 |
| Vitamin E Acetate/Tocopheryl Acetate | 2.00 |
| Cremophor RH 410/PEG-40 Hydrogenated Castor Oil | 1.00 |
| Perfume | q.s. |
| | |

| **B** | |
|---|---|
| Pemulen TR-1 | |
| / Acrylates/C10-30 Alkyl Acrylate Crosspolymer | 0.30 |
| Carbopol 940 / Carbomer | 0.20 |
| 10% extracted mixture in Dioctyl Glycol | 5.00 (Granula Ltd) |
| Edeta BD /EDTA | 0.20 |
| Water | 75.30 |
| | |

| **C** | |
|---|---|
| Sodium Hydroxide | 0.20 |

### Production

Dissolve phase A. Stir phase B into phase A whilst homogenizing, then neutralize with phase C and homogenize again.
Viscosity: approx. 5 500 mPa s (Haake Viscotester VT-02)
pH-value: approx. 9.1

### Example 21

### Sunscreen foam

The Sunscreen Foam contains also various organic compounds (Octyl Methoxycinnamate, Octyl Triazone and 4-Methylbenzylidene Camphor) which have protective properties against UVA and UVB radiation (absorbing UV radiation in critical wavelength and also trapping free radicals induced by UV radiation). Mixture of phenols was extracted from particles of knot wood of spruce into pentylene glycol. The amount of mixture of phenolic compounds in final composition was 0.05 wt%. This mixture of phenolic compounds contributes in capturing free radicals induced by UVA radiation and also capturing free radicals induced by particles of these organic UV-protective compounds.

| **Trade name/ INCI** | **wt %** |
|---|---|
| **A** | |
| Cremophor A 25/Ceteareth-25 | 5.00 |
| Palmitic Acid | 2.00 |
| Finsolv TN/Alkyl Benzoate | 5.00 |
| Witconol APM/PPG-3 Myristyl Ether | 5.00 |
| Uvinul MC 80/Octyl Methoxycinnamate | 6.00 |
| Uvinul T 150/Octyl Triazone | 0.50 |
| Uvinul MBC 95/4-Methylbenzylidene Camphor | 1.00 |
| | |

| **B** | |
|---|---|
| 10% Extracted mixture in Pentylene Glycol | 5.00 |
| Water | 70.30 |
| | |

| **C** | |
|---|---|
| Triethanolamine | 0.20 |
| | |

| **D** | |
|---|---|
| Perfume | q.s. |

### Production

Heat phases A and B separately to about 80° C. Stir phase B into phase A whilst homogenizing. Stir in phase C and rehomogenize. Cool to about 40° C, add phase D and homogenize again.
Filling: 90% active ingredient
10% propane/butane 3.5 bar (20°C).

### Example 22

### Animal feed

Into soybean feed, there was added by spraying a raw extract, with an alcohol content of 90-95 wt%, and compound mixture 5- 10 wt%. The compound mixture was obtained by extracting either a lignan mixture obtained from Norway spruce knotwood by extraction with alcohol, or mixed extract obtained from Norway spruce and pine knotwood by extraction with alcohol, which mixed extract included a compound mixture containing lignans and stilbenes.

In the examples 3-22, the term extracted mixture or knot extract refers to a (poly)phenolic compounds containing compound mixture according to the invention, which mixture is obtained by extracting stem knotwood chips in alcohol. A powdered mixture in turn refers to a corresponding pulverized mixture, which is obtained by pulverizing stem knotwood chips. Mixed extracts of pine and spruce contained 1 part by volume of extracted mixture from pine, and 2 parts by volume of extracted mixture from spruce. Powdered mixtures contained 1 part by weight pulverized pine and 2 parts by weight pulverized spruce.

### Appendix 1

### Figure 1

Figure 1 illustrates different phenolic compounds that can be obtained by a hydrophilic extraction of wood material originated to stem knotwood or bark of pine.

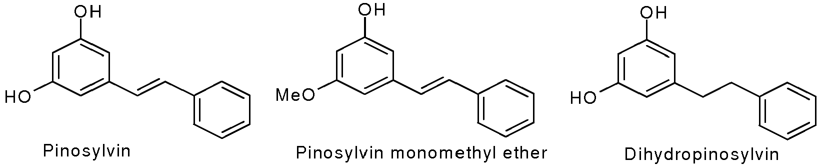

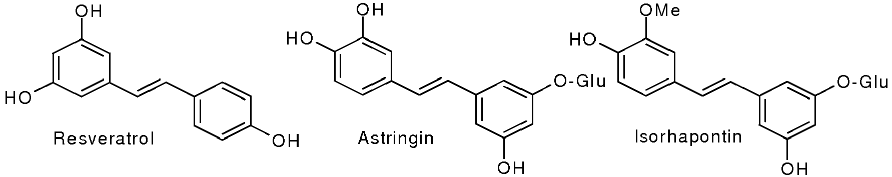

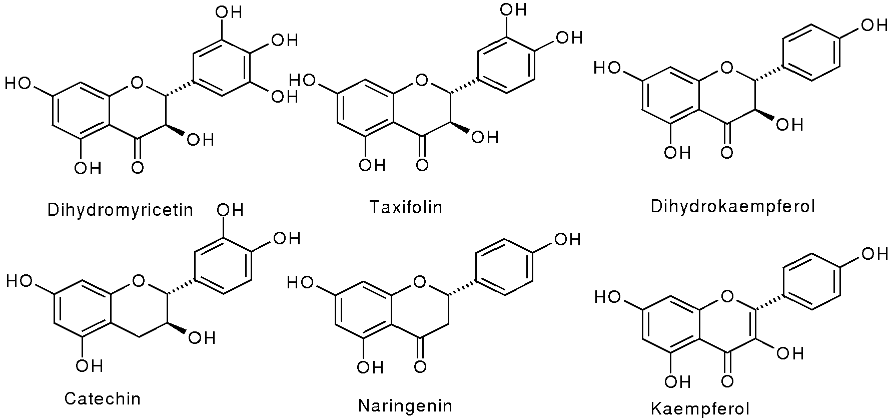

### Appendix 2

### Figure 1 continued

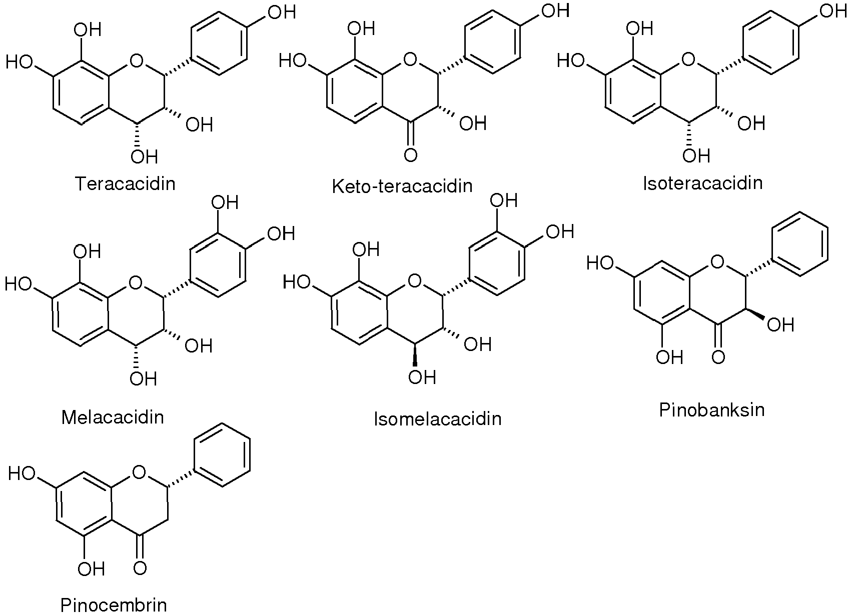

### Appendix 3

### Figure 2

Figure 2 illustrates the different phenolic and polyphenolic compounds contained in the bark of betula pendula (birch tree).

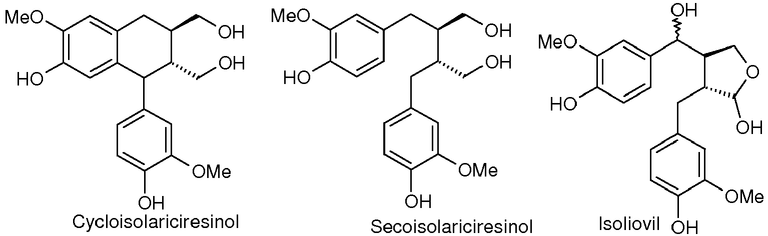

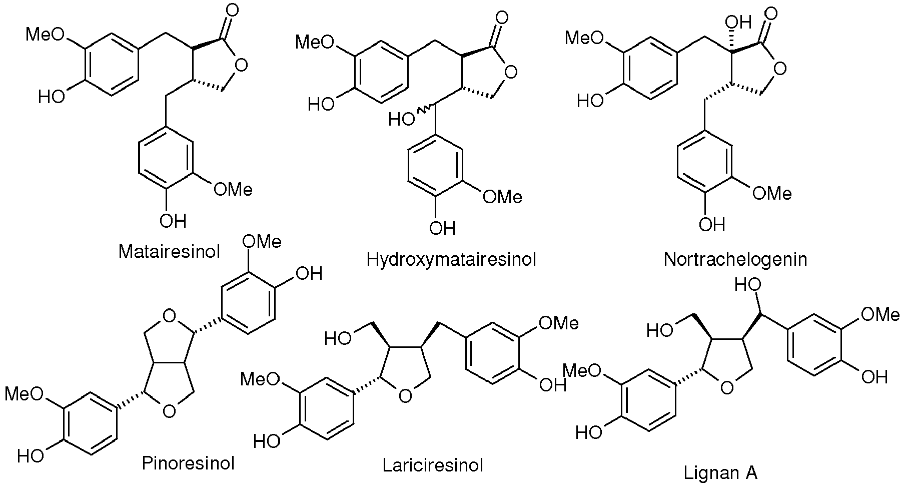

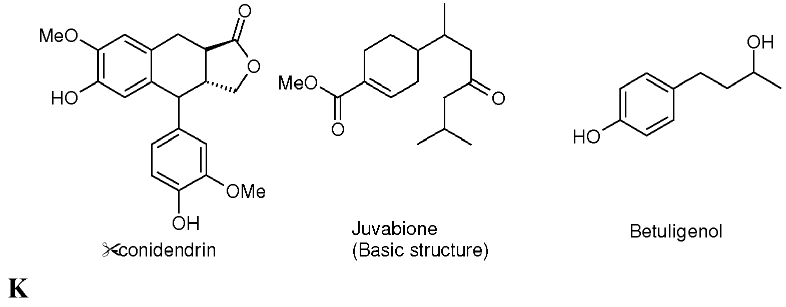

### Appendix 4

**Table 5A**

| | |
|---|---|
| Antioxidative capacity of the lipids in polyphenols-bearing unpurified extracts obtained in hydrophilic extraction from stem knotwood or wood bark, as expressed in terms of extract concentration EC50 µg/L, which inhibits 50% of the peroxidation of lipids. | |

| stem knotwood obtained extracts | EC50 µg/L |
|---|---|
| Acacia crassicarpa | 19 |
| Abies pectinata | 21 |
| Picea glauca | 24 |
| Acasia mangium | 24 |
| Tsuga canadensis | 27 |
| Picea sitchensis | 28 |
| Tsuga heterophylla | 28 |
| Eucalyptus globulus | 57 |
| Abies lasiocarpa | 59 |
| Populus gradidentata | 61 |
| Pinus resinosa | 61 |
| Fagus sylvatica | 91 |
| Populus tremuloidis | 135 |
| Pinus strobes | 159 |
| Populus tremula | 317 |
| | |

| extracts obtained from wood bark | EC50 µg/L |
|---|---|
| Picea abies | 49 |
| Betula pendula | 81 |
| Pycnogenol | 84 |
| Pseudotsugamenziensii | 84 |
| Thuja occidentalis | 131 |
| Pinus banksiana | 143 |
| Populus tremula | 213 |
| Abies lasiocarpa | 316 |

### Appendix 5

**Table 5B**

| | |
|---|---|
| Comparison of the antioxidative capacity of the lipids in pure polyphenol extracts isolated from certain wood materials, as expressed by the extract concentration EC50 µg/L, which inhibits 50 % of the peroxidation of the lipids. | |

| Compound | EC50 µg/L |
|---|---|
| Cyclolariciresinol | 17 |
| Pinoresinol | 20 |
| Melacasidine | 36 |
| Secoisolariciresinol¹ | 37 |
| Taxifolin¹ | 46 |
| Pinosylvin | 50 |
| Teracasidine | 50 |
| Nortrachelogenin¹ | 53 |
| Hydroxymatairesinol¹ | 58 |
| Matairesinol ¹ | 99 |
| Lariciresinol¹ | 126 |
| Dihydrokaempferol | 488 |
| Pinosembrin | 1135 |

| | |
|---|---|
| 1: Willför et al,. J. Agric. Food. Chem, 51. 26 (2003). | |

Tables 5C and 5D further represent the free radicals capturing capacity of certain pure polyphenols and unpurified polyphenol extracts with respect to peroxide radicals.

### Appendix 6

**Table 5C.**

| | | |
|---|---|---|
| Peroxide radicals /(mmol) capturing capacity of unpurified polyphenols-bearing extracts obtained in hydrophilic extraction from stem knotwood or wood bark, as expressed in capacity per gram of extract. | | |

| Wood material, extracts obtained from stem knotwood | capturing mmol/g | capacity |
|---|---|---|
| Acacia crassicarpa | 21 | |
| Eucalyptus globulus | 7.8 | |
| Picea glauca | 7.8 | |
| Abies pectinata | 6.8 | |
| Tsuga canadensis | 6.8 | |
| Acasia mangium | 6.8 | |
| Tsuga heterophylla | 5.8 | |
| Larix lariciana | 5.8 | |
| Larix sibirica | 5.8 | |
| Picea mariana | 5.8 | |
| Picea sitchensis | 4.9 | |
| Pinus sylvestris | 4.9 | |
| Thuja plicata | 3.9 | |
| Populus gradidentata | 3.9 | |
| Fagus sylvatica | 2.9 | |
| Abies lasiocarpa | 2.7 | |
| Pinus resinosa | 2.7 | |
| Pinus banksiana | 1.9 | |
| Pinus strobus | 1.1 | |
| Populus tremuloidis | 0.39 | |
| Populus tremula | 0.29 | |
| | | |
| Wood material, | | |
| wood bark | capturing capacity | |
| obtained extracts | mmol/g | |
| Pseudotsuga menziensii | 4.9 | |

### Appendix 7

| | |
|---|---|
| Pycnogenol | 4.9 |
| Pinus banksiana | 3.1 |
| Betula pendula | 2.9 |
| Picea abies | 2.9 |
| Thuja occidentalis | 1.9 |
| Abies lasiocarpa | 0.58 |
| Populus tremula | 0.29 |

**Table 5D**

| Comparison of the peroxide radicals (mmol) capturing capacity of certain pure polyphenol extracts isolated from wood material, as expressed per gram of extract. | |
|---|---|
| Compound | capturing capacity mmol/g |
| Melacasidine | 20 |
| Taxifolin¹ | 16 |
| Cyclolariciresinol | 12 |
| Secoisolariciresinol¹ | 8.5 |
| Pinoresinol | 7.8 |
| Tetra casidin | 7.8 |
| Nortrachelogenin¹ | 5.9 |
| Hydroxymatairesinol¹ | 5.6 |
| Matairesinol¹ | 2.9 |
| Lariciresinol¹ | 2.7 |
| Dihydrokaempferol | 0.78 |
| Pinosylvin | 0.78 |
| Pinosembrin | 0.49 |

| | |
|---|---|
| 1: Willför et al,. J. Agric. Food. Chem., 51, 7600-7606 (2003). | |

## Claims

1. A method for producing a cosmetic composition, a food industry composition, an animal feed composition, a technical composition or a packing material composition that is administered to a mammal or gets into contact with a mammal, wherein there are added to the composition to be prepared: a carrier agent such as a solvent, with either hydrophobic or hydrophilic nature, selected according to the target of usage; a first effective agent, which is selected from the group consisting of: a micro-organisms growth inhibiting agent, an UV protection agent, an antioxidant and a free radical trapper; a compound mixture 0.1 - 5 wt% from the total weight of said composition, wherein the compounds of compound mixture has properties for modifying the effect of the first effective agent in the target of usage, whereby in the composition, there is included
- a first effective agent defined above,
- a carrier agent, conventionally used in a cosmetic composition, a food industry composition, an animal feed composition, a technical composition or a packing material composition, as well as possible auxiliary agents,
- a compound mixture which is obtained by pulverizing wood material from two different wood species and/or by extracting the possibly pulverized wood material from two different wood species, so that said mixture contains at least two different compounds selected from the group consisting of: lignans according to general formula IA 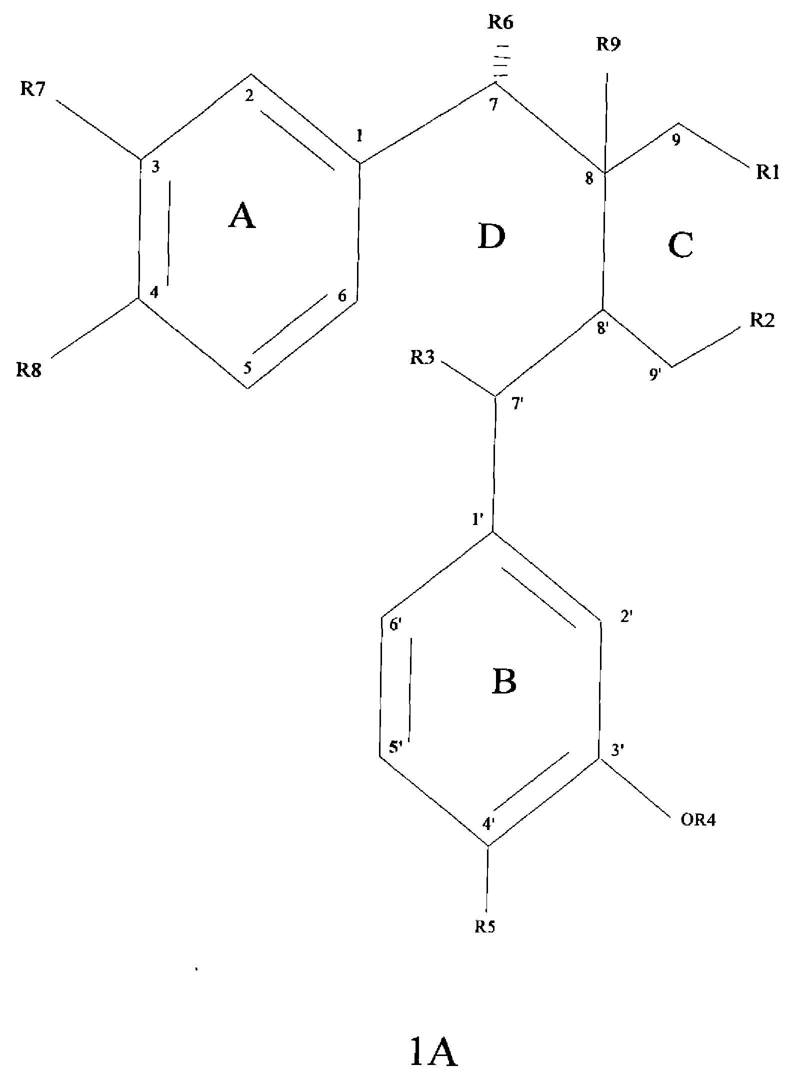
wherein
R1 or R2 denote, irrespective of each other, residue selected from the groups hydrogen, OH or =O,
or either one of the residues R1 or R2 denotes the oxygen atom -O- bound to carbons 9 and 9', and now forms with carbons 8, 8', 9, 9' a 5-membered oxygenous heterocyclic ring C,
R3 denotes hydrogen or residue selected from the group OH, =O, or it forms a bond to the carbon 6, so that the carbons 6, 1, 7, 8, 8', 7' form a cyclohexane ring that is condensed with the phenyl ring A, and possibly also a ring with C,
R4 denotes hydrogen or methyl,
R5 denotes hydrogen or residue selected from the groups OH and OCH₃,
R6 denotes hydrogen or hydroxy,
R7 and R8 denote, irrespective of each other, hydrogen or residue selected from the groups OH and OCH₃.
R9 denotes H or in case of nortrachelogenin R9 denotes OH
or lignans of general formula IB 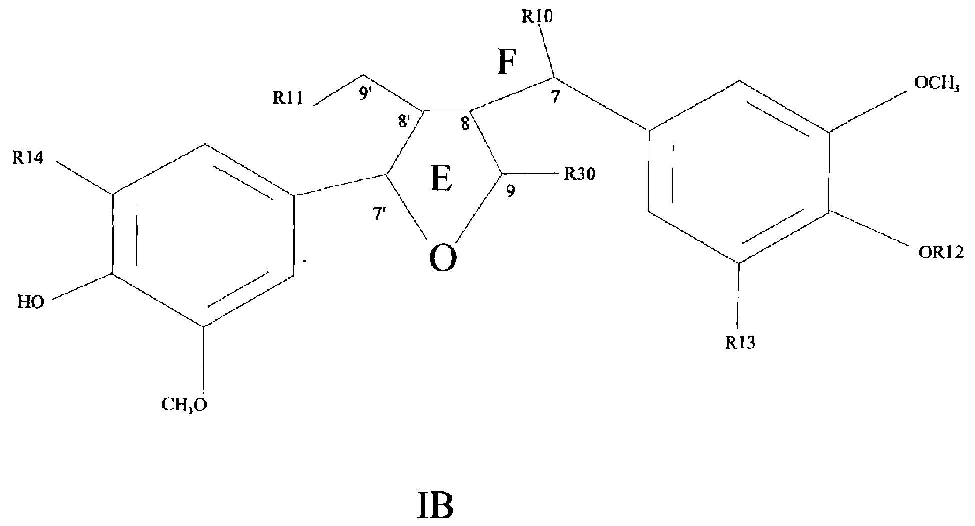 wherein
R10 denotes hydrogen or hydroxy
R11 denotes hydroxy or oxygen, which is bound by a bond to the carbons 7 and 9', forming now an oxygenous non-aromatic 5-membered heterocyclic ring (tetrahydrofuran) F with the carbons 7, 8, 8', 9', which ring is condensed in the hetero ring E (tetrahydrofuran) at the carbons 8, 8',
R12 denotes hydrogen or methyl,
R13 denotes hydrogen or methoxy,
R14 denotes hydrogen or methoxy,
R30 denotes hydrogen or group =O and
stilbenes according to general formula II 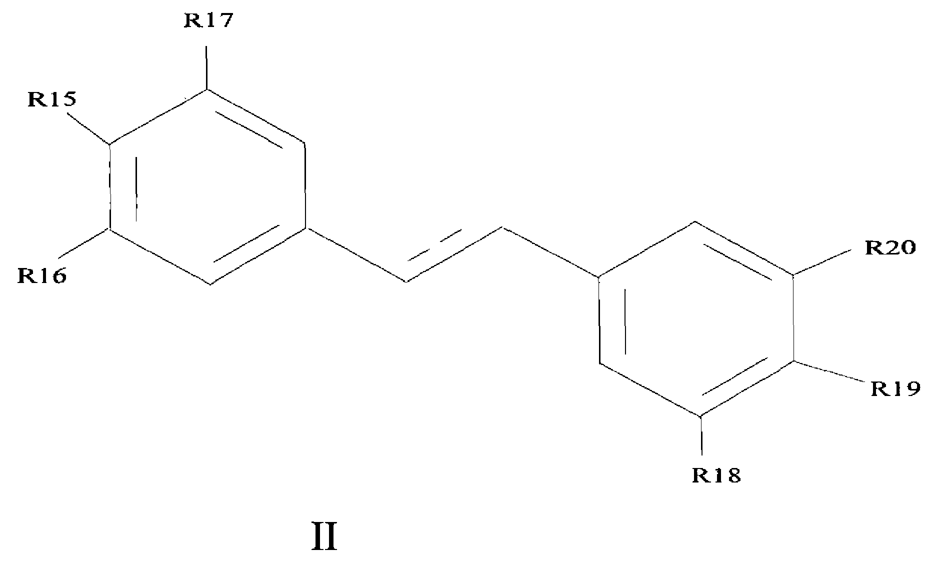 wherein
R15 denotes hydrogen or hydroxy
R16 denotes residue selected from the groups H, OH, OCH₃,
R17 denotes residue selected from the groups OH or OCH₃,
R18 and R19 denote, irrespective of each other, hydrogen or hydroxy,
R20 denotes residue selected from the groups hydrogen, OGlu and
juvabiones according to general formula III, 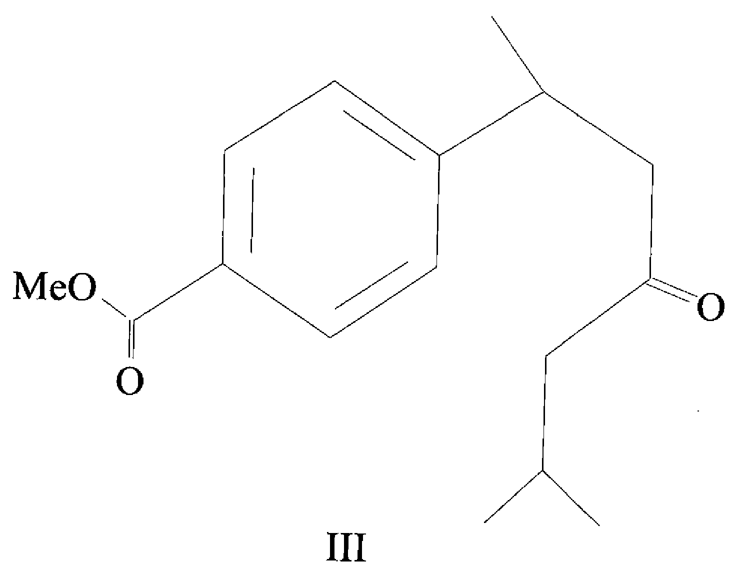 and flavonoids according to general formula IV 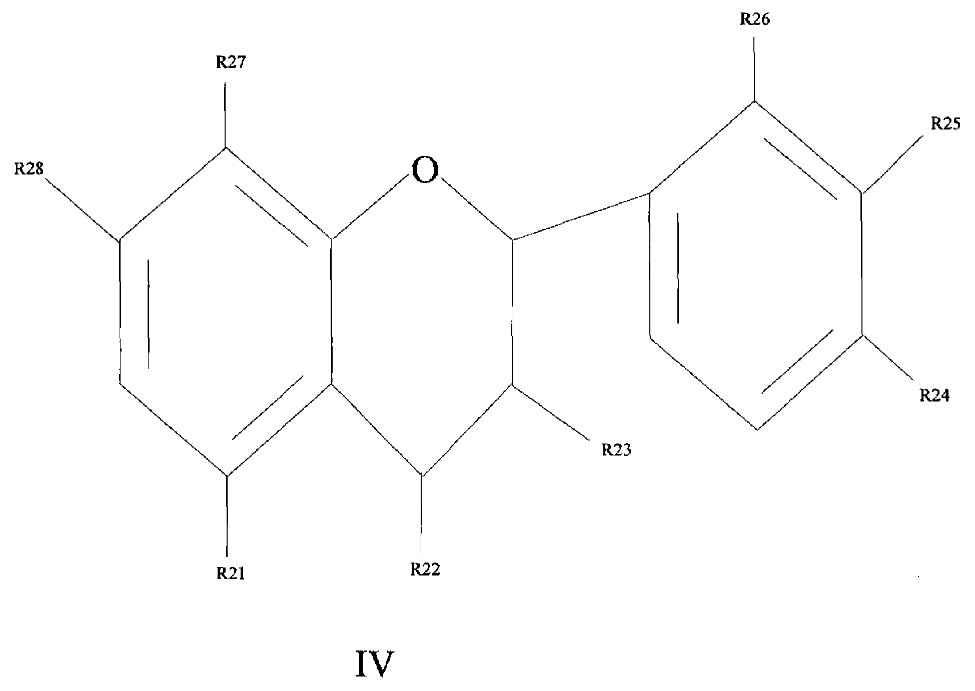 wherein
R21 denotes residue selected from the groups H, OH,
R22 denotes residue selected from the groups H, OH, =O,
R23 denotes residue selected from the groups H, OH
R24, R25, R26 denote, irrespective of each other, hydrogen or hydroxy,
R28 and R27 denote, irrespective of each other, hydrogen or hydroxy,
and betulin and its derivatives (betulinic acid, betuloinic acid or betulonic acid), said compound mixture also containing oligomers of said lignans, stillbenes, juvabiones or flavonoides, providing, however, that the compound mixture contains lignans according to IA or IB, or their ether or ester derivatives or stereoisomers, particularly 7-hydroxymatairesinol or secoisolariciresinol, cycloisolariciresinol, anhydrosecoisolariciresinol, α-conidendrin, α-conidendric acid, isohydroxymatairesinol, or their ether and ester derivatives and stereoisomers for 50-99.9 wt%; stilbenes according to formula II, particularly pinosylvin or its ester or ether derivatives for 0.1 - 70 wt%; oligomers of lignans according to formula IA or IB, of stilbenes according to formula II, of juvabiones according to formula III or of flavonoids according to formula IV for 1 - 31 wt%; providing, however, that the mixture contains at least one compound, selected from among the following group: 7-hydroxymatairesinol, secoisolariciresinol, cycloisolariciresinol, anhydroseco-isolariciresinol, α-conidendrin, α-conidendric acid, isohydroxymatairesinol and stilbenes according to formula II, as well as their ether and ester derivatives and stereoisomers,
characterized thereof that
- the compounds of said compound mixture do not irritate the skin in a so-called single patch test,
- the cytotoxicity of compound mixture, measured as the cytotoxicity of compounds of compound mixture, dissolved in ethanol with a HaCat cell culture after 24 incubations, is kept lower than the cytotoxicity of 0.02 - 0.1 wt% BHT dissolved in ethanol in the same incubation conditions, preferably lower than the cytotoxicity of 0.01 - 0.05 wt% BHT dissolved in ethanol in the same incubation conditions.

2. A method according to claim 1, **characterized in that** the oligomers are oligolignans.

3. A method according to claim 1 or 2, **characterized in that** the compound mixture is a lignan mixture containing at least two different lignan compounds, said lignan mixture being obtained by extracting wood material from two different wood species, preferably wood knot material or stemwood material from adjacent to knots, by an organic hydrophilic solvent, preferably an alcohol-based organic solvent.

4. A method according to any of the claims 1-3, **characterized in that** the pulverized compound mixture, obtained by pulverizing wood material or compound mixture extracted in a solvent, is used as such, without refining, in the production of the composition.

5. A method according to claim 4, **characterized in that** the compound mixture is obtained by extracting material from at least two different wood species by a solution containing alkyl carbonyls, such as alcohols or ketones wherein the compound mixture is preferably obtained by extracting the wood material of pine and spruce (species), and that the compound mixture contains both stilbenes and lignans, 50 - 99.9% of the lignans being obtained from spruce knot material or spruce stemwood material adjacent to knots, and 0.1 - 50% of the lignans being obtained from pine knot material or pine stemwood material adjacent to knots.

6. A method according to claim 5 **characterized in that** the compound mixture contains at least hroxymatairesinol, secoisolariciresinol, conidendrin, lariciresinol, liovile and other lignans while the other lignans containing mainly oligolignans.

7. A method according to any of the preceding claims, **characterized in that** the compound mixture contains at least two different compounds, selected from the following group:
Lignans:
- matairesinol, hydroxymatairesinol, oxomatairesinol, didemethyl matairesinol, isohydroxymatairesinol, epi-isohydroxymatairesinol and their stereoisomers, among which let us particularly point out hydroxymatairesinol stereoisomers 7S, 8R; 8'R-hydroxymatairesinol and 7R, 8R, 8'R-allohydroxymatairesinol, and their stereoisomers and ester or ether derivatives,
- secoisolariciresinol, isolariciresinol, lariciresinol, pinoresinol, dimethyl secoisolariciresinol, 7-hydroxysecoisolariciresinol, cyclolariciresinol, and their stereoisomers and ester or ether derivatives,
- nortrachelogenin and its stereoisomers and ester or ether derivatives,
- enterolactone and its stereoisomers and ester or ether derivatives,
- conidendrin, α-conidendrin, α-conidendric acid, conidendric acid, and their ester or ether derivatives,
- lignan A and its stereoisomers and ester or ether derivatives,
- liovile and its stereoisomers and ester or ether derivatives;
Juvabiones:
- juvabiones and their stereoisomers and ester or ether derivatives;
Stilbenes:
- pinosylvin, dihydropinosylvin, pinosylvin monomethyl ether, dihydropinosylvin monomethyl ether, resveratrol, astringin, isorhapontine, and their stereoisomers and ester or ether derivatives,
Flavonoids:
- pinosembrin, catechin, pinobanxin, kaempferol, dihydrokaempferol, taxifolin, naringenin, teracasidine, ketoteracasidine, isoteracasidine, melacasidine, isomelacasidine and their stereoisomers and ester or ether derivatives,
- as well as the glycosidized forms of these polyphenolic compounds and their oligomers, such as trimers and tetramers, and
- betulin, betulonic acid, betulinic acid, betuloinic acid or their ester derivatives.

8. A method according to claim 1, **characterized in that** the compound mixture is manufactured by extracting wood material first with an organic lipophilic solvent, such as hydrocarbon, and then with an organic hydrophilic solvent, such as an organic lower alkyl carbonyl compound, said hydrophilic solvent forming part of the solvent system of the composition or semifinished product.

9. A method according to claim 8, **characterized in that** in the composition, there also is included an aqueous solvent.

10. A method according to claim 1, **characterized in that** in the composition to be produced, there is included, as the first effective agent, an organic or inorganic UV protector agent that has a certain in vitro protective factor against UV radiation, and that in said composition, there also is included a compound mixture mentioned in claim 1 that increases the *in vivo* protective factor against UV radiation of the composition, with respect to the *in vitro* protective factor.

11. A method according to claim 10, **characterized in that** in the composition, there also is included a synthetic or natural antioxidant whereby the antioxidant is preferably selected from among the following groups:
- agents naturally occurring on mammal skin,
- synthetic antioxidants,
- vitamins, preferably A, B, C, D and E vitamins,
- microbicidic agents that have antioxidative effects,
- enzymes, preferably the enzyme Q10,
- antioxidants obtained from plants, for example green tea,
- melamine and synthetic melamine-like agents,
- antioxidant occurring in green tea,
- antioxidants occurring in the fruits of the eucalyptus tree.

12. A method according to claim 1, **characterized in that** in the composition, there is included as the first effective agent a coated, finely divided inorganic UV protector agent, in the coating of which there is included the compound mixture mentioned in claim 1, having antioxidative and/or free-radical capturing properties, so that it is capable of inhibiting the induction of free radicals from particles originating from inorganic UV protector agents whereby the mentioned inorganic finely divided UV protector agent is preferably titanium oxide, iron oxide, zinc oxide or cerium oxide, the metal oxide particles of which are coated with a silica compound or with a metal oxide, such as aluminum oxide.

13. A method according to claim 12, **characterized in that** the inorganic finely divided coated UV protector agent is dispersed in a liquid medium, such as isononyl-isononanoate, prior to its inclusion in the composition.

14. A method according to claim 1, **characterized in that** the first effective agent to be included in the composition is a natural antioxidant such as a vitamin, and that in addition, in the composition there is included a compound mixture, mentioned in claim 1 that has a higher temperature and UV light resistance than said natural antioxidant.

15. A method according to any of the preceding claims, **characterized in that** the composition is a homogeneous mixture, such as a homogeneous solution, a colloidal dispersion such as a paste-like solution, an emulsion, a microemulsion, a nanoemulsion or a heterogeneous mixture such as a suspension whereby as the surface active agent, the composition or semifinished product contains preferably a tenside or an emulsifier, and the surface active agent is preferably selected from among the following group: cationic surface active agent, anionic surface active agent, amphoteric surface active agent, non-ionogenic surface active agent, cetearyl pyridium chloride, bentsalkonium chloride, cetearyl glycoside, lower alcoxilated glycosides, micelle-forming agents, lecithin.

16. A method according to claim 1, **characterized in that** in the composition, there also is included betulin, betulinic acid, betulonic acid or betuloinic acid or their ester derivatives, obtained from birch bark.

17. The method according to claim 1 for producing a cosmetic composition containing:
- as the first effective agent, antioxidants such as vitamins,
- the compound mixture mentioned in claim 1,
- colloidal W/O or O/W carrier,
- emulsifier,
- additives such as inorganic UV protector agents, viscosity regulator.

18. An intermediate product to be used in the method according to claim 1, **characterized in that** it contains:
- the compound mixture mentioned in claim 1, that is added in the coating of the UV protector agent,
- colloidal W/O or O/W carrier,
- emulsifier,
- inorganic finely divided UV protector agent, where the inorganic finely divided UV protector agents are coated with a silica compound or with a metal oxide, such as aluminum oxide,

19. An intermediate product to be used in the method according to claim 1, **characterized in that** it contains:
- liquid W/O or O/W carrier,
- a UV protector agent imitating the effect of natural melanin on the skin, where the inorganic finely divided UV protector agents are coated with a silica compound or with a metal oxide,
- the compound mixture mentioned in claim 1 that is added in the coating of the UV protector agent,
- emulsifier.

## Patentansprüche

1. Verfahren zum Herstellen einer kosmetischen Zusammensetzung, einer Zusammensetzung aus der Nahrungsmittelindustrie, einer Tierfutterzusammensetzung, einer technischen Zusammensetzung oder einer Packungsmaterialzusammensetzung, die an ein Säugetier verabreicht wird oder mit einem Säugetier in Kontakt kommt, wobei zu der Zusammensetzung, die hergestellt werden soll, hinzugefügt werden: ein Trägermittel wie etwa ein Lösemittel mit entweder einer hydrophoben oder hydrophilen Natur, das gemäß dem Ziel des Gebrauchs ausgewählt wird; ein erster Wirkstoff, der ausgewählt ist aus der Gruppe, die besteht aus: einem Mittel, der das Wachstum von Mikroorganismen hemmt, einem Wirkstoff zum UV-Schutz, einem Antioxidans und aus einem Fänger für freie Radikale; aus einer Verbindungsmischung, die 0,1 bis 5 Gew.% des Gesamtgewichts der Zusammensetzung ausmacht, wobei die Verbindungen der Verbindungsmischung Eigenschaften aufweisen, um die Wirkung des ersten Wirkstoffs in dem Ziel des Gebrauchs zu modifizieren, wobei in der Zusammensetzung enthalten ist:
- ein erster Wirkstoff wie oben definiert,
- ein Trägermittel, das herkömmlicherweise in einer kosmetischen Zusammensetzung, einer Zusammensetzung aus der Nahrungsmittelindustrie, einer Tierfutterzusammensetzung, einer technischen Zusammensetzung oder einer Packungsmaterialzusammensetzung verwendet wird, sowie mögliche Hilfsstoffe,
- eine Verbindungsmischung, die erhalten wird, indem Holzmaterial aus zwei unterschiedlichen Holzarten pulverisiert wird und/oder indem das möglicherweise pulverisierte Holzmaterial aus zwei unterschiedlichen Holzarten extrahiert wird, so dass die Mischung mindestens zwei unterschiedliche Verbindungen enthält, die ausgewählt sind aus der Gruppe, die besteht aus: Lignanen gemäß der allgemeinen Formel IA: 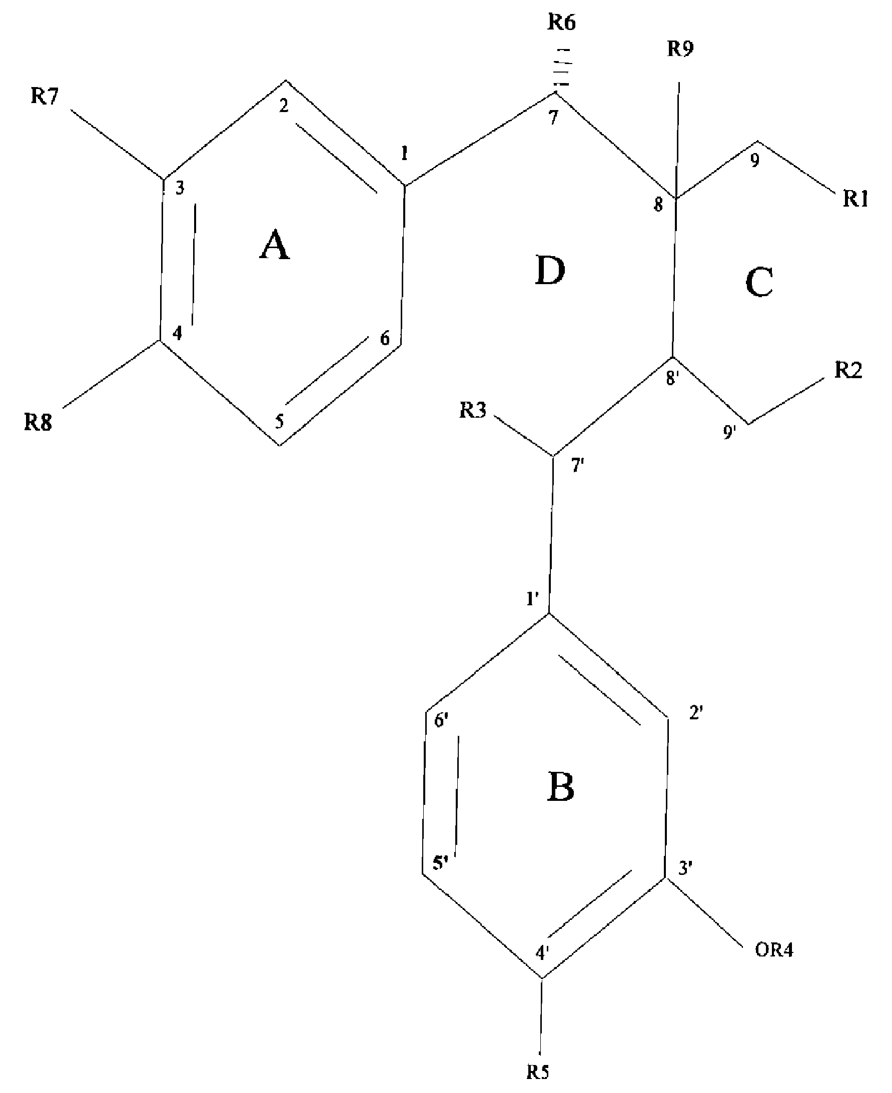 wobei
R1 oder R2 unabhängig voneinander einen Rest bezeichnen, der ausgewählt ist aus den Gruppen Wasserstoff, OH oder =0,
oder einer der Reste R1 oder R2 das Sauerstoffatom -0-bezeichnet, das an den Kohlenstoff an der Stelle 9 und 9' gebunden ist, und jetzt mit den Kohlenstoffatomen 8, 8', 9, 9' einen 5-gliedrigen, sauerstoffhaltigen heterozyklischen Ring C bildet,
R3 Wasserstoff oder einen Rest bezeichnet, der ausgewählt ist aus der Gruppe OH, =0, oder eine Bindung an den Kohlenstoff 6 bildet, so dass die Kohlenstoffatome 6, 1, 7, 8, 8', 7' einen Cyclohexanring, der mit dem Phenylring A kondensiert ist, und möglicherweise auch einen Ring mit C bilden,
R4 Wasserstoff oder Methyl bezeichnet,
R5 Wasserstoff oder einen Rest bezeichnet, der ausgewählt ist aus den Gruppen OH und OCH₃,
R6 Wasserstoff oder Hydroxy bezeichnet,
R7 und R8 unabhängig voneinander Wasserstoff oder einen Rest bezeichnen, der ausgewählt ist aus den Gruppen OH und OCH₃,
R9 H bezeichnet oder wobei R9 im Falle von Nortrachelogenin OH
oder Lignane von der folgenden allgemeinen Formel IB bezeichnet: 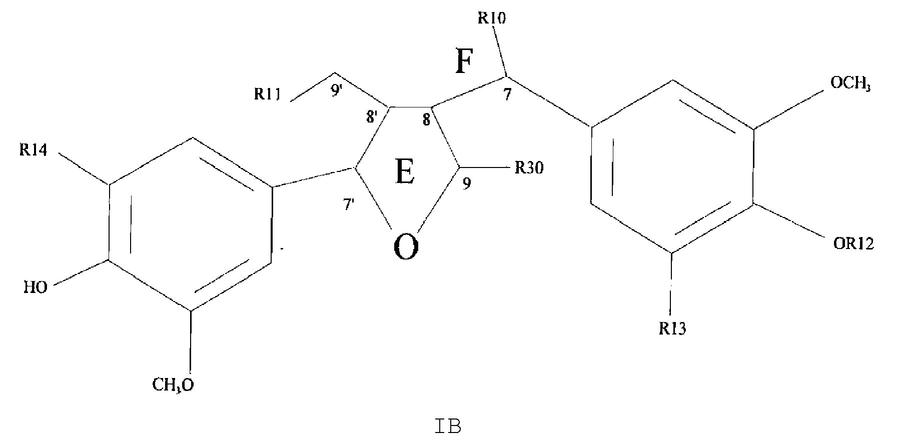 wobei
R10 Wasserstoff oder Hydroxy bezeichnet,
R11 Wasserstoff oder Sauerstoff bezeichnet, der durch eine Bindung an die Kohlenstoffatome an der Stelle 7 und 9' gebunden ist, die jetzt einen sauerstoffhaltigen, nicht-aromatischen, 5-gliedrigen heterozyklischen Ring (Tetrahydrofuran) F mit den Kohlenstoffatomen 7, 8, 8', 9' bilden, wobei der Ring in dem Heteroring E (Tetrahydrofuran) an den Kohlenstoffatomen 8, 8' kondensiert ist,
R12 Wasserstoff oder Methyl bezeichnet,
R13 Wasserstoff oder Methoxy bezeichnet,
R14 Wasserstoff oder Methoxy bezeichnet,
R30 Wasserstoff oder eine Gruppe =0 bezeichnet und
Stilbene gemäß der folgenden allgemeinen Formel II: 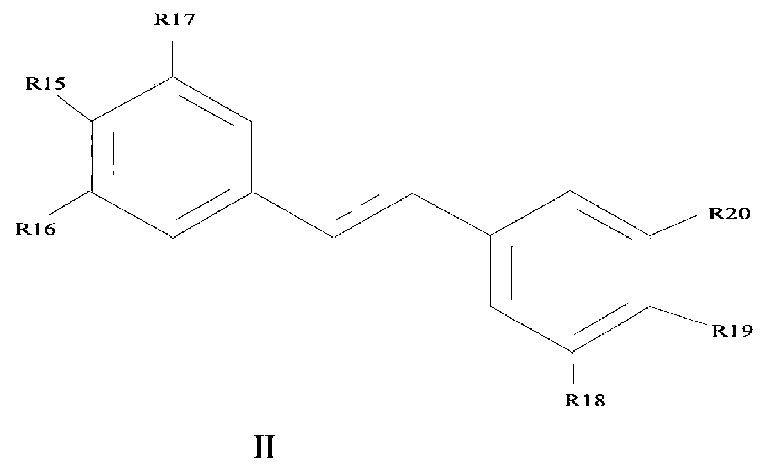 wobei
R15 Wasserstoff oder Hydroxy bezeichnet,
R16 einen Rest bezeichnet, der ausgewählt ist aus den Gruppen H, OH, OCH₃,
R17 einen Rest bezeichnet, der ausgewählt ist aus den Gruppen OH oder OCH₃,
R18 und R19 unabhängig voneinander Wasserstoff oder Hydroxy bezeichnen,
R20 einen Rest bezeichnet, der ausgewählt ist aus den Gruppen Wasserstoff, OGlu und
Juvabione gemäß der folgenden allgemeinen Formel III: 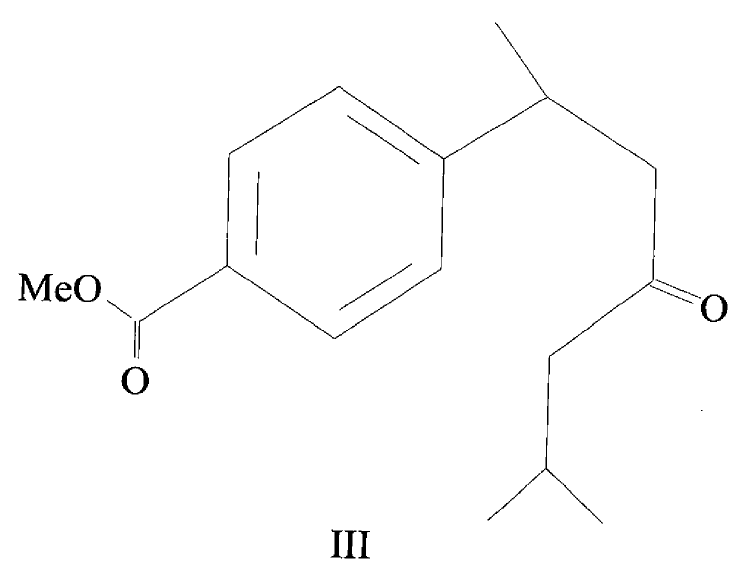 und aus Flavonoiden gemäß der folgenden allgemeinen Formel IV: 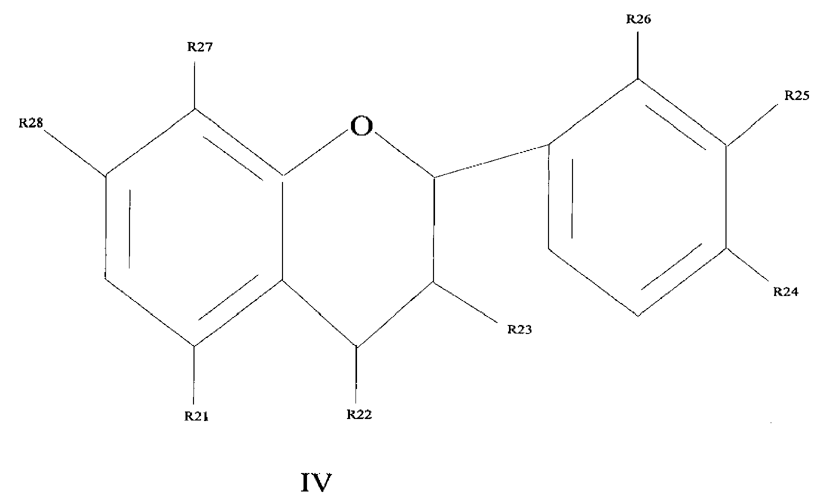 wobei
R21 einen Rest bezeichnet, der ausgewählt ist aus den Gruppen H, OH,
R22 einen Rest bezeichnet, der ausgewählt ist aus den Gruppen H, OH, =0,
R23 einen Rest bezeichnet, der ausgewählt ist aus den Gruppen H, OH,
R24, R25, R26 unabhängig voneinander Wasserstoff oder Hydroxy bezeichnen,
R28 und R27 unabhängig voneinander Wasserstoff oder Hydroxy bezeichnen,
und Betulin und seine Derivate (Betulinsäure, Betuloinsäure oder Betulonsäure), wobei die Verbindungsmischung auch Oligomere der Lignane, der Stilbene, Juvabione oder der Flavonoiden enthält, vorausgesetzt jedoch, dass die Verbindungsmischung Lignane gemäß IA oder IB oder deren Ether- oder Esterderivate oder Stereoisomere enthält, insbesondere 7-Hydroxymatairesinol oder Secoisolariciresinol, Cycloisolariciresinol, Anhydrosecoisolariciresinol, α-Conidendrin, α-Conidendrinsäure, Isohydroxymatairesinol oder deren Ether- und Esterderivate und Stereoisomere für 50 bis 99,9 Gew.%; Stilbene gemäß Formel II, insbesondere Pinosylvin oder seine Ester- oder Etherderivate für 0,1 bis 70 Gew.%; Oligomere von Lignanen gemäß Formel IA oder IB, von Stilbenen gemäß Formel II, von Juvabionen gemäß Formel III oder von Flavonoiden gemäß Formel IV für 1 bis 31 Gew.%; vorausgesetzt jedoch, dass die Mischung mindestens eine Verbindung enthält, die aus der folgenden Gruppe ausgewählt ist: 7-Hydroxymatairesinol, Secoisolariciresinol, Cycloisolariciresinol, Anhydroseco-isolariciresinol, α-Conidendrin, α-Conidendrinsäure, Isohydroxymatairesinol und Stilbene gemäß Formel II ebenso wie deren Ether- und Esterderivate und Stereoisomere,
gekennzeichnet davon, dass
- die Verbindungen der Verbindungsmischung die Haut in einer so genannten einzelnen Verträglichkeitsprüfung mit einem Testpflaster (Patchtest) nicht reizen,
- die Cytotoxizität der Verbindungsmischung, die als die Cytotoxizität der Verbindungen der Verbindungsmischung, die in Ethanol mit einer HaCat-Zellkultur nach 24 Inkubationen aufgelöst ist, gemessen wird, niedriger gehalten wird als die Cytotoxizität von 0,02 bis 0,1 Gew.% BHT, die in Ethanol unter denselben Inkubationsbedingungen aufgelöst ist, bevorzugt niedriger als die Cytotoxizität von 0,01 bis 0,05 Gew.% BHT, die in Ethanol unter denselben Inkubationsbedingungen aufgelöst ist.

2. Verfahren nach Anspruch 1, **dadurch gekennzeichnet, dass** die Oligomere Oligolignane sind.

3. Verfahren nach Anspruch 1 oder 2, **dadurch gekennzeichnet, dass** die Verbindungsmischung eine Lignanmischung ist, die mindestens zwei verschiedene Lignanverbindungen enthält, wobei die Lignanmischung erhalten wird, indem Holzmaterial aus zwei unterschiedlichen Holzarten, bevorzugt Holzastmaterial oder Stammholzmaterial aus der Nachbarschaft zu Ästen, durch ein organisches hydrophiles Lösemittel, vorzugsweise ein alkoholbasiertes organisches Lösemittel, extrahiert wird.

4. Verfahren nach einem der Ansprüche 1 bis 3, **dadurch gekennzeichnet, dass** die pulverisierte Verbindungsmischung, die durch ein Pulverisieren von Holzmaterial erhalten wird, oder die Verbindungsmischung, die in einem Lösemittel extrahiert wird, als eine solche ohne Veredelung in der Produktion der Zusammensetzung verwendet wird.

5. Verfahren nach Anspruch 4, **dadurch gekennzeichnet, dass** die Verbindungsmischung erhalten wird, indem Material von mindestens zwei verschiedenen Holzarten durch eine Lösung, die Alkylcarbonyle wie etwa Alkohole oder Ketone enthält, extrahiert wird, wobei die Verbindungsmischung vorzugsweise durch ein Extrahieren des Holzmaterials von einer Kiefer und von einer Fichte (Holzarten) erhalten wird, und dass die Verbindungsmischung sowohl Stilbene als auch Lignane enthält, wobei 50 bis 99,9 % der Lignane aus einem Holzmaterial von Fichtenholzästen oder aus einem Fichtenstammholzmaterial, das benachbart zu Ästen liegt, erhalten werden, und wobei 0,1 bis 50 % der Lignane aus einem Holzmaterial von Kiefernholzästen oder aus einem Kiefernstammholzmaterial, das benachbart zu Ästen liegt, erhalten werden.

6. Verfahren nach Anspruch 5, **dadurch gekennzeichnet, dass** die Verbindungsmischung mindestens Hydroxymatairesinol, Secoisolariciresinol, Conidendrin, Lariciresinol, Liovile und andere Lignane enthält, während die anderen Lignane vorwiegend Oligolignane enthalten.

7. Verfahren nach einem der vorhergehenden Ansprüche, **dadurch gekennzeichnet, dass** die Verbindungsmischung mindestens zwei verschiedene Verbindungen enthält, die aus der folgenden Gruppe ausgewählt sind:
Lignane:
- Matairesinol, Hydroxymatairesinol, Oxomatairesinol, Didemethyl-matairesinol, Isohydroxymatairesinol, Epi-isohydroxymatairesinol und ihre Stereoisomere, unter denen besonders die Hydroxymatairesinol-Stereoisomere 7S, 8R; 8'R-Hydroxymatairesinol und 7R, 8R, 8'R-Allohydroxymatairesinol und ihre Stereoisomere und Ester- oder Etherderivate hervorzuheben sind,
- Secoisolariciresinol, Isolariciresinol, Lariciresinol, Pinoresinol, Dimethylsecoisolariciresinol, 7-Hydroxysecoisolariciresinol, Cycloisolariciresinol und ihre Stereoisomere und Ester- oder Etherderivate,
- Nortrachelogenin und seine Stereoisomere und Ester- oder Etherderivate,
- Enterolacton und seine Stereoisomere und Ester- oder Etherderivate,
- Conidendrin, α-Conidendrin, α-Conidendrinsäure, Conidendrinsäure und ihre Ester- oder Etherderivate,
- Lignan A und seine Stereoisomere und Ester- oder Etherderivate,
- Liovil und seine Stereoisomere und Ester- oder Etherderivate;
Juvabione:
- Juvabione und deren Stereoisomere und Ester- oder Etherderivate;
Stilbene
- Pinosylvin, Dihydropinosylvin, Pinosylvinmonomethylether, Dihydropinosylvinmonomethylether, Resveratrol, Astringin, Isorhapontin und deren Stereoisomere und Ester- oder Etherderivate,
Flavonoide:
- Pionosembrin; Catechin, Pinobanxin, Kaempferol, Dihydrokaempferol, Taxifolin, Naringenin, Teracasidin, Ketoteracasidin, Isoteracasidin, Melacasidin, Isomelacasidin und deren Stereoisomere und Ester- oder Etherderivate,
- sowie die glycosidierten Formen von diesen Polyphenolverbindungen und deren Oligomere wie etwa Trimere und Teramere, und
- Betulin, Betulonsäure, Betulinsäure, Betuloinsäure oder deren Esterderivate.

8. Verfahren nach Anspruch 1, **dadurch gekennzeichnet, dass** die Verbindungsmischung hergestellt wird, indem Holzmaterial zuerst mit einem organischen lipophilen Lösemittel wie etwa Kohlenwasserstoff, und dann mit einem organischen hydrophilen Lösemittel wie etwa eine organische niedere Alkylcarbonylverbindung extrahiert wird, wobei das hydrophile Lösemittel einen Teil des Lösemittelsystems der Zusammensetzung oder des halbfertigen Produkts bildet.

9. Verfahren nach Anspruch 8, **dadurch gekennzeichnet, dass** in der Zusammensetzung auch ein wässriges Lösemittel enthalten ist.

10. Verfahren nach Anspruch 1, **dadurch gekennzeichnet, dass** in der Zusammensetzung, die hergestellt werden soll, als der erste Wirkstoff ein organischer oder anorganischer UV-Schutzwirkstoff enthalten ist, der einen gewissen in vitro Schutzfaktor gegen UV-Strahlung aufweist, und dass in der Zusammensetzung auch eine Verbindungsmischung enthalten ist, die in Anspruch 1 erwähnt ist und die den in vitro Schutzfaktor gegen UV-Strahlung der Zusammensetzung in Bezug auf den in vitro Schutzfaktor erhöht.

11. Verfahren nach Anspruch 10, **dadurch gekennzeichnet, dass** in der Zusammensetzung auch ein synthetisches oder natürliches Antioxidans enthalten ist, wobei das Antioxidans vorzugsweise aus den folgenden Gruppen ausgewählt ist:
- Mittel, die natürlicherweise auf der Haut eines Säugetiers auftreten,
- synthetische Antioxidanzien,
- Vitamine, vorzugsweis die Vitamine A, B, C, D und E,
- mikrobielle Mittel, die antioxidative Wirkungen aufweisen,
- Enzyme, vorzugsweise das Enzym Q10,
- Antioxidanzien, die aus Pflanzen, zum Beispiel aus dem grünen Tee erhalten werden,
- Melamin und melaminähnliche Mittel,
- Antioxidans, das in dem grünen Tee auftritt,
- Antioxidanzien, die in den Früchten des Eukalyptusbaumes auftreten.

12. Verfahren nach Anspruch 1, **dadurch gekennzeichnet, dass** in der Zusammensetzung als der erste Wirkstoff ein beschichteter, fein verteilter anorganischer UV-Schutzwirkstoff enthalten ist, in dessen Beschichtung die in Anspruch 1 erwähnte Verbindungsmischung enthalten ist, die antioxidative Eigenschaften und/oder Eigenschaften zum Einfangen freier Radikale aufweist, so dass sie in der Lage ist, die Induktion freier Radikale von Partikeln, die von anorganischen UV-Schutzwirkstoffen stammen, zu hemmen, wobei der erwähnte anorganische, fein verteilte UV-Schutzwirkstoff vorzugsweise Titanoxid, Eisenoxid, Zinkoxid oder Ceroxid ist, deren Metalloxidpartikel mit einer Siliciumdioxidverbindung oder mit einem Metalloxid wie etwa Aluminiumoxid beschichtet sind.

13. Verfahren nach Anspruch 12, **dadurch gekennzeichnet, dass** der anorganische, fein verteilte, beschichtete UV-Schutzwirkstoff vor seinem Einschluss in die Zusammensetzung in einem flüssigen Medium wie etwa Isononyl-isononanoat verteilt ist.

14. Verfahren nach Anspruch 1, **dadurch gekennzeichnet, dass** der erste Wirkstoff, der in der Zusammensetzung enthalten sein soll, ein natürliches Antioxidans wie etwa ein Vitamin ist, und dass zusätzlich in der Zusammensetzung eine Verbindungsmischung, die in Anspruch 1 erwähnt ist, enthalten ist, die eine höhere Temperaturresistenz und eine höhere UV-Lichtresistenz als das natürliche Antioxidans aufweist.

15. Verfahren nach einem der vorhergehenden Ansprüche, **dadurch gekennzeichnet, dass** die Zusammensetzung eine homogene Mischung wie etwa eine homogene Lösung, eine kolloidale Dispersion wie etwa eine pastenähnliche Lösung, eine Emulsion, eine Mikroemulsion, eine Nanoemulsion oder eine heterogene Mischung wie etwa eine Suspension, wobei als der grenzflächenaktive Stoff die Zusammensetzung oder das halbfertige Produkt vorzugsweise ein Tensid oder einen Emulgator enthält, und wobei der grenzflächenaktive Stoff vorzugsweise aus der folgenden Gruppe ausgewählt ist: kationischer grenzflächenaktiver Stoff, anionischer grenzflächenaktiver Stoff, amphoterer grenzflächenaktiver Stoff, nicht-ionogener grenzflächenaktiver Stoff, Cetearylpyridiumchlorid, Bentsalkoniumchlorid, Cetarylglycosid, niedere alcoxilierte Glycoside, Micelle bildende Mittel, Lecithin.

16. Verfahren nach Anspruch 1, **dadurch gekennzeichnet, dass** in der Zusammensetzung auch Betulin, Betulinsäure, Betulonsäure oder Betuloinsäure oder deren Esterderivate, die von der Birkenrinde gewonnen werden, enthalten sind.

17. Verfahren nach Anspruch 1 zum Herstellen einer kosmetischen Zusammensetzung, die enthält:
- als den ersten Wirkstoff Antioxidanzien wie etwa Vitamine,
- die Verbindungsmischung, die in Anspruch 1 erwähnt ist,
- einen kolloidalen W/O- oder O/W-Träger,
- einen Emulgator,
- Zusatzstoffe wie etwa anorganische UV-Schutzwirkstoffe, einen Viskositätsregulator.

18. Zwischenprodukt, das in dem Verfahren nach Anspruch 1 verwendet werden soll, **dadurch gekennzeichnet, dass** es enthält:
- die Verbindungsmischung, die in Anspruch 1 erwähnt ist und die der Beschichtung des UV-Schutzwirkstoffs hinzugefügt ist,
- einen kolloidalen W/O- oder O/W-Träger,
- einen Emulgator
- einen anorganischen, fein verteilten UV-Schutzwirkstoff, wobei die anorganischen, fein verteilten UV-Schutzwirkstoffe mit einer Siliciumdioxidverbindung oder mit einem Metalloxid wie etwa Aluminiumoxid oder mit einem Metalloxid wie etwa Aluminiumoxid beschichtet sind.

19. Zwischenprodukt, das in dem Verfahren nach Anspruch 1 verwendet werden soll, **dadurch gekennzeichnet, dass** es enthält:
- einen flüssigen W/O- oder O/W-Träger,
- einen UV-Schutzwirkstoff, der die Wirkung von natürlichem Melanin auf der Haut nachahmt, wobei die anorganischen, fein verteilten UV-Schutzwirkstoffe mit einer Siliciumdioxidverbindung oder mit einem Metalloxid beschichtet sind,
- die Verbindungsmischung, die in Anspruch 1 erwähnt ist und die der Beschichtung des UV-Schutzwirkstoffs hinzugefügt ist,
- einen Emulgator.

## Revendications

1. Procédé de production d'une composition cosmétique, d'une composition industrielle de nourriture, d'une composition technique ou d'une composition de matériel d'emballage qui est administrée à un mammifère ou entre en contact avec un mammifère, dans lequel la composition à être préparée : un agent de transport tel qu'un solvant, soit de nature hydrophobe soit de nature hydrophile, choisi selon la cible d'utilisation ; un premier agent efficace, qui est choisi parmi le groupe constitué : d'un agent inhibant la croissance de microorganismes, d'un agent de protection anti-UV, d'un antioxydant et d'un piégeur de radicaux libres ; un mélange de composés compris entre 0,1 et 5% en poids à partir du poids total de ladite composition, dans lequel les composés du mélange de composés possèdent des propriétés pour modifier l'effet du premier agent efficace dans la cible d'utilisation, selon lequel dans la composition, est inclus
- un premier agent efficace tel que défini ci-dessus,
- un agent de transport, utilisé conventionnellement dans une composition cosmétique, une composition industrielle de nourriture, une composition d'alimentation pour animaux, une composition technique ou une composition de matériel d'emballage, de même que des agents auxiliaires,
- un mélange de composés qui est obtenu en pulvérisant du matériau en bois à partir de deux espèces de bois différentes et/ou en extrayant le matériau en bois possiblement pulvérisé à partir de deux espèces de bois différentes, de sorte que ledit mélange contient au moins deux composés différents choisis parmi le groupe constitué de : ligands selon la formule générale IA 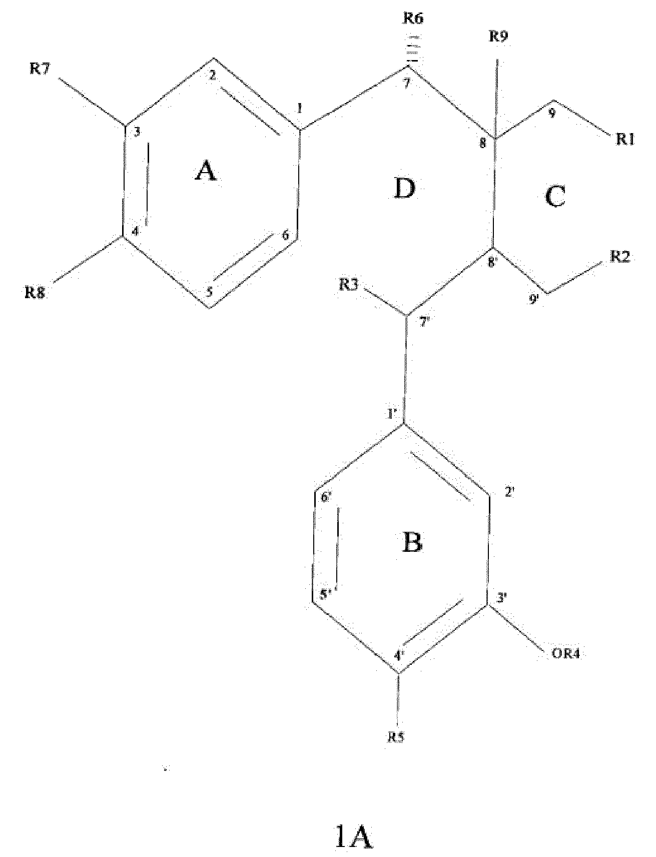 dans laquelle
R1 ou R2 désignent, indépendamment l'un de l'autre, un résidu choisi parmi les groupes hydrogène, OH ou =0,
ou soit un des résidus R1 ou R2 désigne la liaison de l'atome d'oxygène -0- aux carbones 9 et 9', et maintenant forme avec les carbones 8, 8', 9, 9' un noyau C hétérocyclique oxygéné à 5 chainons,
R3 désigne l'hydrogène ou un résidu choisi parmi le groupe OH, =0, ou il forme une liaison au carbone 6, de sorte que les carbones 6, 1, 7, 8, 8', 7' forment un cycle cyclohexane qui est condensé avec un noyau phényle A, et éventuellement aussi un noyau avec C,
R4 désigne l'hydrogène ou méthyle,
R5 désigne l'hydrogène ou un résidu choisi parmi les groupes OH et OCH₃,
R6 désigne l'hydrogène ou hydroxy,
R7 et R8 désignent, indépendamment l'un de l'autre, l'hydrogène ou un résidu choisi parmi les groupes OH et OCH₃,
R9 désigne H ou en cas de nortrachélogénine R9 désigne OH
ou des ligands de formule générale IB 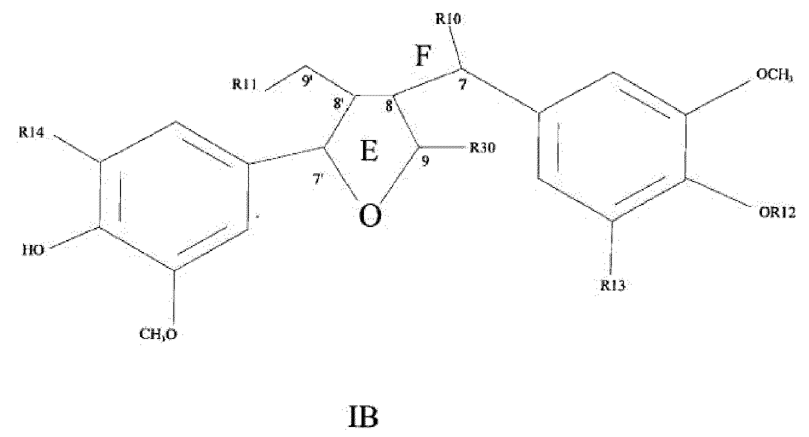 dans laquelle
R10 désigne l'hydrogène ou hydroxy
R11 désigne hydroxy ou l'oxygène, qui est lié par une liaison aux carbones 7 et 9', formant maintenant un noyau (tétrahydrofurane) F hétérocyclique à 5 chainons non aromatique oxygéné avec les carbones 7, 8, 8', 9', lequel noyau est condensé dans le noyau E (tétrahydrofurane) hétéro aux carbones 8,8',
R12 désigne l'hydrogène ou méthyle,
R13 désigne l'hydrogène ou méthoxy,
R14 désigne l'hydrogène ou méthoxy,
R30 désigne l'hydrogène ou un groupe =0
et des stilbènes selon la formule générale II 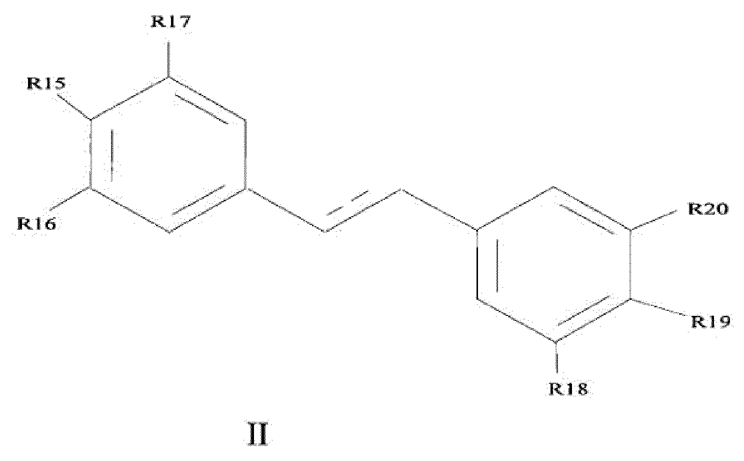 dans laquelle
R15 désigne l'hydrogène ou hydroxy
R16 désigne un résidu choisi parmi les groupes H, OH, OCH₃,
R17 désigne un résidu choisi parmi les groupes OH ou OCH₃,
R18 et R19 désignent, indépendamment l'un de l'autre, l'hydrogène ou hydroxy,
R20 désigne un résidu choisi parmi les groupes hydrogène, OGlu
et des juvabiones selon la formule générale III, 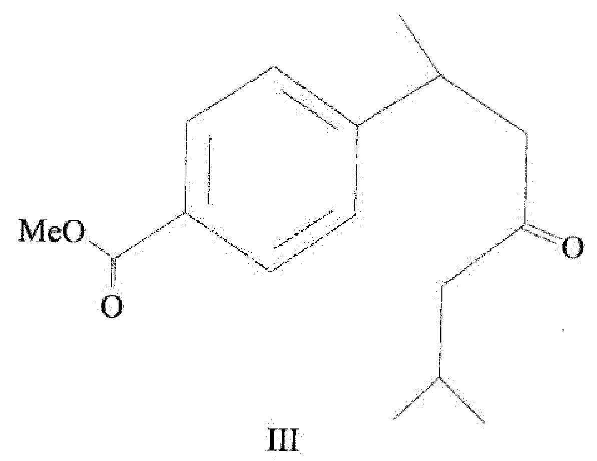 et des flavonoïdes selon la formule générale IV 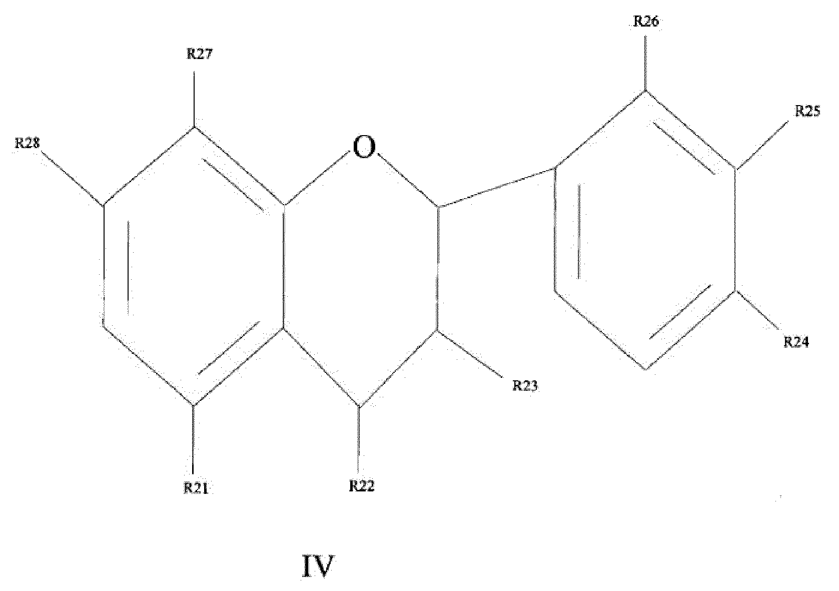 dans laquelle
R21 désigne un résidu choisi parmi les groupes H, OH,
R22 désigne un résidu choisi parmi les groupes H, OH, =0,
R23 désigne un résidu choisi parmi les groupes H, OH,
R24, R25, R26 désignent, indépendamment l'un de l'autre, l'hydrogène ou hydroxy,
R28 et R27 désignent, indépendamment l'un de l'autre, l'hydrogène ou hydroxy,
et la bétuline et ses dérivés (acide bétulinique, acide bétuloïnique ou acide bétulonique), ledit mélange de composés contenant également des oligomères desdits ligands, stilbènes, juvabiones ou flavonoïdes, à condition, cependant, que le mélange de composés contient des ligands selon IA ou IB, ou leur dérivés éther ou ester ou stéréoisomères, particulièrement 7-hydroxymatairésinol ou sécoisolaricirésinol, cycloisolaricirésinol, anhydrosécoisolaricirésinol, α-conidendrine, acide α-conidendrique, isohydroxymatairésinol, ou leur dérivés éther ou ester ou stéréoisomères pour de 50 à 99,9% en poids ; des stilbènes selon la formule II, en particulier la pinosylvine ou ses dérivés éther ou ester pour de 0,1 à 70% en poids ; des oligomères de ligands selon la formule IA ou IB, des stilbènes selon la formule II, des juvabiones selon la formule III ou des flavonoides selon la formule IV pour 31% en poids ; à condition, cependant, que le mélange de composés contient au moins un composé, choisi parmi le groupe suivant : 7-hydroxymatairésinol, sécoisolaricirésinol, cycloisolaricirésinol, anhydrosécoisolaricirésinol, α-conidendrine, acide α-conidendrique, isohydroxymatairésinol et des stilbènes selon la formule II, ainsi que leur dérivés éther et ester et stéréoisomères,
**caractérisé en ce que**
- les composés dudit mélange de composés n'irritent pas la peau chez un soi-disant test épicutané unique,
- la cytotoxicité du mélange de composés, mesurée comme la cytotoxicité des composés du mélange de composés, dissout dans de l'éthanol avec une culture cellulaire HaCat après 24 incubations, est maintenue inférieure à la cytotoxicité de 0,02 à 0,1% en poids de BHT dissout dans de l'éthanol dans les mêmes conditions d'incubation, de préférence inférieure à la cytotoxicité de 0,01 à 0,05% en poids de BHT dissout dans de l'éthanol dans les mêmes conditions d'incubation.

2. Procédé selon la revendication 1, **caractérisé en ce que** les oligomeres sont des oligolignans.

3. Procédé selon la revendication 1 ou 2, **caractérisé en ce que** le mélange de composés est un mélange de lignans contenant au moins deux composés lignans différents, ledit mélange de ligans étant obtenu en extrayant du matériau en bois à partir de deux espèces de bois différentes, de préférence du matériau en noeuds de bois ou du matériau en bois de fût d'adjacent aux noeuds, par un solvant hydrophile organique, de préférence un solvant organique à base d'alcool.

4. Procédé selon l'une quelconque des revendications 1 - 3, **caractérisé en ce que** le mélange pulvérisé de composés, obtenu en pulvérisant le matériau en bois ou le mélange de composés extrait dans un solvant, est utilisé en tant que tel, sans raffinage, dans la production de la composition.

5. Procédé selon la revendication 4, **caractérisé en ce que** le mélange de composés est obtenu en extrayant du matériau à partir d'au moins deux espèces de bois différentes à l'aide d'une solution contenant des alkyl carbonyle, tels que des alcools ou cétones dans laquelle le mélange de composés est de préférence obtenu en extrayant le matériau en bois de pin et d'épicéa (espèces), et que le mélange de composés contient à la fois des stilbènes et de lignans, de 50 à 99,9% des lignans étant obtenus à partir du matériau en noeuds d'épicéa ou du matériau du bois de fût d'épicéa adjacent aux noeuds, et de 0,1 à 50% des lignans étant obtenus à partir du matériau en noeuds de pin ou du matériau en bois de fût de pin adjacent aux noeuds.

6. Procédé selon la revendication 5, **caractérisé en ce que** le mélange de composés contient au moins hydroxymatairésinol, sécoisolaricirésinol, conidendrine, laricirésinol, liovile et d'autres lignans alors que les autres lignans contiennent principalement des oligolignans.

7. Procédé selon l'une quelconque des revendications précédentes, **caractérisé en ce que** le mélange de composés contient au moins deux composés différents, choisis parmi le groupe suivant :
Lignans :
- matairésinol, hydroxymatairésinol, oxomatairésinol, didéméthyl matairésinol, isohydroxymatairésinol, épiisohydroxymatairésinol et leur stéréoisomères, parmi lesquels laissez nous signaler en particulier les stéréoisomères 7S, 8R de l'hydroxymatairésinol ; le 8'R-hydroxymatairésinol et 7R, 8R, 8'R-allohydroxymatairésinol, et leur stéréoisomères et dérivés ester ou éther,
- sécoisolaricirésinol, isolaricirésinol, laricirésinol, pinorésinol, diméthyl sécoisolaricirésinol, 7-hydroxysécoisolaricirésinol, cyclolaricirésinol, et leur stéréoisomères et dérivés ester ou éther,
- nortrachélogénine et ses stéréoisomères et dérivés ester ou éther,
- entérolactone et ses stéréoisomères et dérivés ester ou éther,
- conidendrine, α-conidendrine, acide α-conidendrique, acide conidendrique et leur dérivés ester ou éther,
- lignan A et ses stéréoisomères et dérivés ester ou éther,
- liovile et ses stéréoisomères et dérivés ester ou éther ;
Juvabiones :
- pinosylvine, dihydropinosylvine, éther monométhylique de pinosyl
- vine, éther monométhylique de dihydropinosylvine, resvératrol, astringine, isorhapontine, et leur stéréoisomères et dérivés ester ou éther, Stilbènes :
- pinosylvine, dihydropinosylvine, éther monométhylique de pinosylvine, éther monométhylique de dihydropinosylvine, resvératrol, astringine, isorhapontine, et leur stéréoisomères et dérivés ester ou éther,
Flavonoïdes :
- pinosembrine, catéchine, pinobanxine, dihydrokaempférol, taxofoline, naringénine, téracasidine, kétotéracasidine, isotéracasidine, mélacasidine, isomélacasidine et leur stéréoisomères et dérivés ester ou éther,
- ainsi que les formes glycosidées de ces composés polyphénoliques et leur oligomères, tels que trimères et tétramères, et
- bétuline, acide bétulinique, acide bétuloïnique ou leur dérivés ester.

8. Procédé selon la revendication 1, **caractérisé en ce que** le mélange de composés est fabriqué en extrayant le matériau en bois premièrement avec un solvant lipophile organique, tel qu'un hydrocarbure, et ensuite avec un solvant hydrophile organique, tel qu'un composé alkyl carbonyle inférieur organique, ledit solvant hydrophile faisant partie du système de solvant de la composition ou du produit semi-fini.

9. Procédé selon la revendication 8, **caractérisé en ce que** dans la composition, un solvant aqueux est également inclus.

10. Procédé selon la revendication 1, **caractérisé en ce que** dans la composition qui doit être produite, est inclus, en tant que premier agent efficace, un agent organique ou inorganique protecteur contre les UV qui possède un certain facteur de protection in vitro contre les rayonnements UV, et que dans ladite composition, est également inclus un mélange de composés mentionné dans la revendication 1 qui augmente le facteur de protection *in vitro* contre les rayonnements UV de la composition, par rapport au facteur de protection *in vitro.*

11. Procédé selon la revendication 10, **caractérisé en ce que** dans la composition, est également inclus un antioxydant synthétique ou naturel selon lequel l'antioxydant est choisi de préférence parmi les groupes suivants :
- des agents existant naturellement sur la peau des mammifères,
- des antioxydants synthétiques,
- des vitamines, de préférence les vitamines A, B, C, D et E,
- des agents microbicides qui possèdent des effets antioxydants,
- des enzymes, de préférence l'enzyme Q10,
- des antioxydants obtenus à partir de plantes, par exemple le thé vert,
- de la mélanine et des agents synthétiques ressemblant à la mélanine,
- un antioxydant existant dans le thé vert,
- des antioxydants existant dans les fruits de l'arbre de l'eucalyptus.

12. Procédé selon la revendication 1, **caractérisé en ce que** dans la composition, est inclus en tant que premier agent efficace un agent inorganique protecteur contre les UV finement divisé, recouvert, dans le revêtement duquel est inclus le mélange de composés mentionné dans la revendication 1, possédant des propriétés antioxydantes ou de capture de radicaux libres, de sorte qu'il est capable d'inhiber l'induction de radicaux libres à partir de particules ayant pour origine les agents inorganiques protecteurs contre les UV selon lequel l'agent inorganique protecteur contre les UV finement divisé est de préférence l'oxyde de titane, l'oxyde de fer, l'oxyde de zinc ou l'oxyde de cérium, les particules d'oxyde de métal duquel sont recouvertes d'un composé de silice ou d'un oxyde de métal, tel que l'oxyde d'aluminium.

13. Procédé selon la revendication 12, **caractérisé en ce que** l'agent inorganique protecteur contre les UV finement divisé, recouvert est dispersé dans un milieu liquide, tel que l'isononyl-isononanoate, avant son inclusion dans la composition.

14. Procédé selon la revendication 1, **caractérisé en ce que** le premier agent efficace qui doit être inclus dans la composition est un antioxydant naturel tel qu'une vitamine, et qu'en plus, dans la composition est inclus un mélange de composés, mentionné dans la revendication 1 qui possède une température et une résistance aux rayons UV supérieure audit antioxydant naturel.

15. Procédé selon l'une quelconque des revendications, **caractérisé en ce que** la composition est un mélange homogène, tel qu'une solution homogène, une dispersion colloïdale telle qu'une solution ressemblant à une pâte, une émulsion, une microémulsion, une nanoémulsion ou un mélange hétérogène tel qu'une dispersion selon laquelle en tant qu'agent actif de surface, la composition ou le produit semi-fini contient de préférence un tensioactif ou un émulsifiant, et l'agent actif de surface est choisi de préférence parmi le groupe suivant : un agent tensioactif cationique, un agent tensioactif anionique, un agent tensioactif amphotère, un agent tensioactif non-ionogène, le chlorure de cétéaryl pyridium, le chlorure de bentsalkonium, le cétéaryl glycoside, des glycosides alcoxylés inférieurs, des agents formant des micelles, la lécithine.

16. Procédé selon la revendication 1, **caractérisé en ce que** dans la composition, est également inclus de la bétuline, de l'acide bétulinique, de l'acide bétulonique ou de l'acide bétuloïnique ou leur dérivés ester, obtenus à partir de l'écorce de bouleau.

17. Procédé selon la revendication 1 pour la production d'une composition cosmétique contenant :
- en tant que premier agent efficace, des antioxydants tels que des vitamines,
- le mélange de composés mentionné dans la revendication 1,
- un transporteur colloïdal W/O ou O/W,
- un émulsifiant,
- des additifs tels que des agents inorganiques protecteurs contre les UV, régulateur de viscosité.

18. Produit intermédiaire destiné à être utilisé dans le procédé selon la revendication 1, **caractérisé en ce qu'**il contient :
- le mélange de composés mentionné dans la revendication 1, qui est ajouté au revêtement de l'agent protecteur contre les UV,
- un transporteur colloïdal W/O ou O/W,
- un émulsifiant,
- un agent inorganique protecteur contre les UV finement divisé, où les agents inorganiques protecteurs contre les UV finement divisés sont recouverts d'un composé de silice ou d'un oxyde de métal, tel que l'oxyde d'aluminium.

19. Produit intermédiaire destiné à être utilisé dans le procédé selon la revendication 1, **caractérisé en ce qu'**il contient :
- un transporteur liquide W/O ou O/W,
- un agent protecteur contre les UV imitant l'effet de la mélanine naturelle sur la peau, où les agents inorganiques protecteurs contre les UV finement divisés sont recouverts d'un composé de silice ou d'un oxyde de métal,
- le mélange de composés mentionné dans la revendication 1, qui est ajouté au revêtement de l'agent protecteur contre les UV,
- un émulsifiant.
